# EUROPEAN PATENT APPLICATION

(11) **EP 4 620 459 A1**
(43) Date of publication of application: **24.09.2025**
(21) Application number: 24855541.9
(22) Date of filing: 20.07.2024
(51) Int. Cl.: A61K 9/36, A61K 31/155, A61P 3/10

(54) **METFORMIN HYDROCHLORIDE ENTERIC-COATED TABLET**

(30) Priority: 22.08.2023 CN 202311065738
(71) Applicant: Yayan (Tianjin) Biomedical Technology Center (Limited Partnership), Tianjin 301721 (CN)
(72) Inventor: GAO, Qian, Tianjin 301721 (CN); DONG, Tao, Tianjin 301721 (CN); WANG, Wei, Tianjin 301721 (CN)
(74) Representative: Danubia Patent & Law Office LLC
(86) International application number: PCT/CN2024/106617
(87) International publication number: WO 2025/039814

(57) **Abstract**

The invention provides a metformin hydrochloride enteric-coated tablet, which is composed of a tablet core containing a therapeutically effective amount of metformin hydrochloride and an enteric coating layer wrapping the tablet core. The dissolution rate of metformin hydrochloride in a pH 4.5 medium within 2 hours is not higher than 15%, and the dissolution rate of metformin hydrochloride in a pH 6.8 medium within 20 minutes is not lower than 85%. The present invention obtains the metformin hydrochloride enteric-coated tablet with the specific drug release characteristics by controlling the disintegration time of the tablet core, the enteric coating material with the specific onset dissolving point and the weight gain in coating. Compared with the common metformin hydrochloride preparation (such as Glucophage) or the metformin hydrochloride enteric-coated tablet with other release characteristics, the tolerability and compliance of the metformin hydrochloride enteric-coated tablet said in the present invention are improved without reducing the hypoglycemic efficacy.

## Description

### TECHNICAL FIELD

The invention provides a metformin hydrochloride enteric-coated tablet, which is a delayed-release preparation. The invention belongs to the field of pharmaceutical preparations.

### BACKGROUND OF THE INVENTION

Metformin is an oral biguanide hypoglycemic drug. Its hydrochloride, namely metformin hydrochloride, is commonly used clinically. In the early days, it was believed that the main mechanism of metformin's hypoglycemic effect was the systemic effect produced after drug absorption, such as inhibiting gluconeogenesis, reducing hepatic glucose output, acting on peripheral tissues (muscle, adipose tissue), promoting glucose utilization and improving insulin sensitivity. Recent studies have found that the intestinal mediated mechanism produced by unabsorbed drugs (such as increasing GLP-1 and PYY levels, affecting intestinal flora and inhibiting intestinal wall cells from taking up glucose) may be more important.

In the diabetes diagnosis and treatment guidelines formulated by many countries and international organizations, metformin hydrochloride is recommended as the first - line drug for controlling hyperglycemia in patients with type 2 diabetes and is the basic drug in combination therapy. Glucophage (metformin hydrochloride tablets) is an original product of metformin, with many years of clinical application experience and sufficient evidence-based medicine. It is the standard treatment drug for type 2 diabetes globally and the most commonly used control drug in clinical trials.

However, some adverse reactions of metformin restrict its use. Some patients cannot tolerate these adverse reactions and have to stop taking the drug. The main adverse reactions of metformin include gastrointestinal reactions, such as diarrhea, nausea, vomiting, abdominal distension, abdominal pain, indigestion, abdominal discomfort, etc., and nervous system adverse reactions, such as dizziness and headache. In addition, it can also cause flu-like symptoms and vitamin B12 absorption disorders, and may also cause some rare but more serious adverse reactions, such as lactic acidosis. Gastrointestinal adverse reactions (such as nausea, abdominal pain, abdominal discomfort, etc.) are the most common adverse reactions of metformin, with an incidence of 20-30%, and are also the most common cause of patient intolerance.

In order to reduce the adverse reactions caused by the use of metformin, there are many studies and reports on its enteric-coated preparations in the prior art. Enteric-coated preparations refer to preparations that do not release or almost do not release the drug in the stomach within a specified time, but can release most or all in a certain part of the intestine after entering the intestine.

Early studies and reports on enteric-coated metformin hydrochloride preparations, such as CN101190179B, only focused on whether the drug was not released or almost not released in 0.1 mol/L hydrochloric acid and whether it was completely released in pH 6.8 phosphate buffer. Later related studies and reports, such as CN101785763B, disclosed enteric-coated sustained-release tablets of metformin hydrochloride, which released about 30% of the drug in 1 hour, about 50% in 3 hours, and more than 85% in 10 hours in pH 6.8 phosphate buffer, claiming that the sustained release of the drug improved bioavailability and avoided the possible toxic side effects caused by excessive plasma concentration in a short period of time. CN102357088A disclosed an enteric-coated metformin hydrochloride preparation that was rapidly released in pH 5.5-6.8 buffers and the release was maintained above 80% even in a pH 5.5 phosphate buffer after 45 minutes, claiming that the drug could avoid adverse reactions and the rapid release characteristics can ensure effective release in intestinal fluid with a pH value of 5.5-6.8 and can more effectively ensure the clinical efficacy of the product. CN106420653A discloses metformin hydrochloride enteric-coated tablets with a dissolution amount of 20-30% in pH 6.8 phosphate buffer in 1 hour, 45-55% in 2 hours and more than 85% in 4 hours, claiming to have low adverse reactions and be suitable for patients with contraindications to metformin. CN111888339A discloses metformin hydrochloride enteric-coated tablets which release more than 85% in pH 4.5-5.0 buffer in only 15-30 minutes and have high release rate, claiming to have improved bioavailability and be bioequivalent to the original drug. The enteric-coated metformin hydrochloride preparations disclosed and reported in these prior arts meet the common requirements that enteric-coated preparations do not release or almost release no drugs in acidic medium of pH 1.0-3.0, and release most or all of the drugs in buffered salt medium of pH 6.8. However, the further release requirements (such as the speed or degree of release in the medium, etc.) and the therapeutic effects achieved by the enteric-coated preparations is vague in the prior arts. Some have rapid release to ensure efficacy and /or safety, while others have sustained release to ensure efficacy and /or safety, which are obviously contradictory to each other and lack further verification.

In addition, ELCELYX THERAPEUTICS, INC. disclosed an enteric-coated preparation of metformin hydrochloride in WO2013103384A, WO2013103919A and WO2014107617A. The preparation is a delayed release preparation (DR) designed to push the drug into the distal small intestine to release the active drug, thereby improving the gastrointestinal tolerance and reducing the adverse events caused by the biguanide compound. WO2013103384A and WO2013103919A disclose a delayed-release formulation having a relative bioavailability reduced by 20% to 60%, preferably 40% to 60%, compared to a conventional formulation, i.e., an immediate -release formulation (IR), having an equal amount of the biguanide compound. WO2014107617A discloses an enteric-coated oral dosage form containing a biguanide compound, wherein the dosage form showed a lag phase in drug release of at least about 5 or 10 minutes after contact with a pH of 6.0, 6.5, 6.8 or 7.0, preferably a lag phase in drug release of at least about 15 or 20 minutes after contact with the desired pH, and more preferably a lag phase in drug release of at least about 25 or 30 minutes after contact with a pH of 6.0, 6.5, 6.8 or 7.0; in a preferred Example, the enteric-coated oral dosage form containing a biguanide compound release less than 15% of the drug after two hours at acid pH and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8.. In another preferred Example, the enteric-coated oral dosage form containing a biguanide compound releases less than 15 %, 10%, or 5% of the biguanide compound in two hours at acidic pH, 30 minutes at pH 5.5, and a lag period of at least ten or fifteen minutes at pH 6.8, releases at least 60 % of the biguanide compound after the lag period and within 60 minutes at pH 6.8, and releases at least 90 % of the biguanide compound within 90 to 120 minutes at pH 6.8 .

The prior art such as WO2014107617A discloses enteric-coated preparations of metformin hydrochloride (delayed-release preparations, DR), which claim that the hypoglycemic efficacy is not less than that of the same dose of ordinary metformin hydrochloride tablets, namely, immediate-release preparations (IR), and even has an increased efficacy. Moreover, compared with IR preparations, DR preparations of metformin hydrochloride reduce the relative bioavailability and systemic exposure of the drug, improve the gastrointestinal tolerance of the drug, and reduce adverse drug events. However, the hypoglycemic efficacy of these studies is evaluated by fasting plasma glucose (FPG) or GLP-1, PYY, etc., which has poor accuracy, especially when the number of cases is small. Therefore, the hypoglycemic efficacy of DR preparations of metformin hydrochloride described is not confirmed clinically. For example, Elcelyx later conducted a 16-week clinical study on metformin hydrochloride DR preparations with an expanded number of cases (see Robert R. Henry et al., Improved glycemic control with minimal systemic metformin exposure: Effects of Metformin Delayed-Release (Metformin DR) targeting the lower bowel over 16 weeks in a randomized trial in subjects with type 2 diabetes, PLOS ONE, published on September 25, 2018, https://doi.org/10.1371/journal.pone.0203946), and used the clinically recognized gold standard glycated hemoglobin (HbA1c) as the evaluation indicator, the results showed that the metformin hydrochloride DR preparation prepared by the technical solution disclosed in the patent document failed to show the expected clinical effect. In the 16-week clinical trial, the best dosing regimen, i.e. giving patients metformin hydrochloride DR every morning, the hypoglycemic efficacy of DR 1500 mg was much lower than that of the control group metformin hydrochloride IR 2000 mg (regular administration according to the instructions, 1000 mg in the morning and 1000 mg in evening), and the reduction in efficacy was greater than the reduction in dosage.

### SUMMARY OF THE INVENTION

In another patent application filed on the same day, it is described that the inventors surprisingly found that the enteric-coated preparation of metformin hydrochloride with the following specific drug release characteristics can produce the best therapeutic effect and the lowest adverse reaction, namely: the dissolution rate of metformin hydrochloride in a pH 4.5 medium within 2 hours is not higher than 15%, and the dissolution rate of metformin hydrochloride in a pH 6.8 medium within 20 minutes is not less than 85%, and more preferably the dissolution rate of metformin hydrochloride in a pH 4.5 medium within 2 hours is not higher than 7.5%, the dissolution rate in pH 6.8 medium is more than 85% within 15 minutes. After clinical trials, the results show that the enteric-coated preparation of metformin hydrochloride with the said drug release characteristics improves the gastrointestinal tolerance of metformin hydrochloride without reducing or even improving the hypoglycemic efficacy, and reduces the adverse reactions caused by the drug. Moreover, the enteric-coated preparation can be taken before meals, after meals or during meals, which improves the patient's compliance with medication. Further pharmacokinetic and in vitro and in vivo correlation studies show that different enteric-coated preparations of metformin hydrochloride with the above-mentioned drug release characteristics are bioequivalent to the preparation of the preferred Example (E1), that is, they have the same efficacy and safety. The content of this patent application is introduced into this application by reference.

Enteric-coated tablets are a common enteric-coated preparation, which are usually coated with an enteric coating on the outside of ordinary tablets. The present invention has found that for metformin hydrochloride enteric-coated tablets there is a close correlation in the disintegration time of tablet core, the onset dissolving point of the enteric coating material (i.e. pH-dependent solubility) and the weight gain in coating. In particular, the metformin hydrochloride enteric-coated tablets prepared by the specific onset dissolving point of the enteric coating material and the specific weight gain in coating have the above-mentioned specific drug release technical requirements, producing the best therapeutic effect and the lowest adverse reactions.

The technical solution of the present invention is as follows:
The invention provides a metformin hydrochloride enteric-coated tablet, which comprising a tablet core containing a therapeutically effective amount of metformin hydrochloride and an enteric coating layer wrapping the tablet core, characterized in that the dissolution rate of metformin hydrochloride in a pH 4.5 medium within 2 hours is not higher than 15%, and the dissolution rate of metformin hydrochloride in a pH 6.8 medium within 20 minutes is not less than 85%. Preferably, the dissolution rate of metformin hydrochloride in a pH 4.5 medium within 2 hours is not higher than 7.5%, and the dissolution rate of metformin hydrochloride in a pH 6.8 medium within 15 minutes is not less than 85%.

Preferably, according to the above-mentioned metformin hydrochloride enteric-coated tablets, characterized in that in the said enteric coating layer the onset dissolving point of the coating material is a pH value of 5.0 to 6.0 and the weight gain in coating is 4.0-12.8 mg/cm². Preferably, the onset dissolving point of the coating material is 5.0 to 6.0 by pH value and the weight gain in coating is 4.8-9.6 mg/cm².

Preferably, as one of the specific Examples of the present invention, in the above-mentioned metformin hydrochloride enteric-coated tablets, characterized in that in the said enteric coating layer the onset dissolving point of the coating material is a pH value of 5.0 and the weight gain in coating is 5.6-12.8 mg/cm².

Preferably, as one of the specific Examples of the present invention, in the above-mentioned metformin hydrochloride enteric-coated tablets, characterized in that in the said enteric coating layer the onset dissolving point of the coating material is a pH value of 5.5 and the weight gain in coating is 4.8-11.2 mg/cm².

Preferably, as one of the specific Examples of the present invention, in the above-mentioned metformin hydrochloride enteric-coated tablets, characterized in that in the said enteric coating layer the onset dissolving point of the coating material is a pH value of 6.0 and the weight gain in coating is 4.0-8.8 mg/cm².

Preferably, in the above-mentioned metformin hydrochloride enteric-coated tablets, the coating material of the enteric coating layer is selected from one or more of the following: hydroxypropyl methylcellulose phthalate (HP-50), methacrylic acid ethyl acrylate copolymer (L100-55), methacrylic acid ethyl acrylate copolymer (L30D-55), hydroxypropyl methylcellulose phthalate (HP-55), hydroxypropyl methylcellulose acetate succinate (HPMCAS-L), methacrylic acid methyl methacrylate copolymer (L100 ; polyacrylic acid resin II), hydroxypropyl methylcellulose acetate succinate (HPMCAS-M), cellulose acetate phthalate (CAP).

Preferably, in the above-mentioned metformin hydrochloride enteric-coated tablets, the disintegration time of the tablet core does not exceed 10 minutes. For example, the disintegration time of the tablet core is 2 to 8 minutes. Preferably, the disintegration time of the tablet core does not exceed 5 minutes. More preferably, the disintegration time of the tablet core is 2-4 minutes.

The above-mentioned enteric-coated metformin hydrochloride tablets of the present invention can be coated with a separation layer on the tablet core before the enteric coating layer as needed. For example, when the weight gain in coating of the enteric coating layer is low, adding a separation coating layer between the tablet core and the enteric coating layer can reduce the amount of enteric material and enhance the enteric effect. For enteric materials with compatibility problems with drugs, adding a separation coating layer can increase the stability of the drug.

Preferably, the above-mentioned metformin hydrochloride enteric-coated tablets of the present invention have a relative bioavailability of (60~80)% compared with ordinary preparations or rapid-release preparations (such as Glucophage) containing the same content of metformin hydrochloride.

As another object of the present invention, a method for reducing or lowering adverse reactions caused by metformin hydrochloride is provided, wherein metformin hydrochloride is prepared into the metformin hydrochloride enteric-coated tablets described above in the present invention.

Preferably, in the above method, the adverse reaction is a digestive tract adverse reaction, such as but not limited to nausea, abdominal pain, abdominal discomfort, etc.

As another object of the present invention, the use of the above-mentioned metformin hydrochloride enteric-coated tablets in the preparation of a medicament for treating a disease is also provided, wherein the disease includes but is not limited to diabetes and obesity.

Enteric-coated tablets are usually prepared by coating an enteric coating on the outside of ordinary tablets. The preparation of the metformin hydrochloride enteric-coated tablets of the present invention, such as the process of preparing the tablet core and the process of coating the tablet core with an enteric coating layer, can be prepared by those skilled in the art according to the general preparation process of enteric-coated tablets in the art. According to the disintegration time of the tablet core, the onset dissolving point of the enteric coating material and the weight gain in coating in the said present invention, especially the selected onset dissolving point of the enteric coating material and the enteric coating weight gain, those skilled in the art can prepare the metformin hydrochloride enteric-coated tablets with the specific drug release technical requirements of the present invention. For enteric-coated tablets with low weight gain in coating, although it is often not easy to meet the acid resistance requirements or the dissolution requirements in a pH 4.5 medium, by controlling appropriate process parameters, for example, checking the liquid supply speed of the peristaltic pump before coating to ensure stable liquid supply, adjusting the atomization pressure and the fan pressure so that the coating liquid has a better atomization state, and strictly controlling humidity during the entire coating process to ensure the density of the coating film, or adding a coating isolation coat, etc., those skilled in the art can prepare the enteric-coated tablets described in the present invention.

In the present invention, "dissolution rate" is also referred to as "release rate", and "dissolution" is also referred to as "release", which have the same meaning in terms of expression. The dissolution or release of a drug can be determined according to the commonly used determination methods in the art, for example, according to the dissolution and release determination method of the first method of General Chapter 0931 of the Chinese Pharmacopoeia (2020 Edition).

In the present invention, the onset dissolving point is a term to describe the pH-dependent solubility of the enteric coating material, that is, the pH value at which the enteric material begins to dissolve. For example, if the pH value of the onset dissolving point of the enteric material is 5.0, it means that the enteric material dissolves at pH ≥ 5.0. For the understanding of the onset dissolving point of the coating material, reference may be made to the description of the solubility of poly(meth)acrylic acid resin on page 534 of the Handbook of Pharmaceutical Excipients (Fourth Edition), 2005, Chemical Industry Press. Different types material dissolve at different pH values, such as Eudragit L100 dissolves at pH > 6, Eudragit S100 dissolves at pH > 7, which is understood as the onset dissolving points of Eudragit L100 and Eudragit S100 are pH 6 and pH 7 respectively. It can also refer to the description of solubility in the table on page 17 of the product manual of hydroxypropyl methylcellulose acetate succinate produced by Shin-Etsu Chemical of Japan. AS-LF and AS-LG dissolve at pH ≥ 5.5, and AS-MF and AS-MG dissolve at pH ≥ 6.0. It can be understood that the onset dissolving points of AS-LF and AS-LG are pH 5.5, and the onset dissolving points of AS-MF and AS-MG are pH 6.0.

The disintegration or disintegration time of the tablet core in the present invention can be determined according to the commonly used measurement methods in the art. For example, it can be determined according to the test method of disintegration time in General Chapter 09 2 1 of the Chinese Pharmacopoeia (2020 Edition).

In the present invention, the pH 4.5 medium is preferably a pH 4.5 acetate buffer, and the pH 6.0 or pH 6.8 medium is preferably a pH 6.0 or pH 6.8 phosphate buffer.

The enteric-coated tablets of the present invention are tablets having the specific drug release characteristics said in the present invention while meeting the general drug release characteristics of enteric-coated preparations. The general drug release characteristics of enteric-coated preparations refer to preparations that do not release or almost do not release drugs in a specified acidic medium (e.g., pH 1.0-3.0), but release most or all of the drugs in a near-neutral medium (e.g., pH 6.0-6.8 phosphate buffer) within a required time.

In the present invention, the dosage or therapeutically effective amount of metformin hydrochloride in a unit preparation, the therapeutic uses of metformin hydrochloride and its adverse reactions, etc., are prior arts known in the art. For example, the prior arts mentioned in the specification, the relevant contents disclosed therein are also introduced into the present application by reference.

It is obtained that the enteric-coated preparation of metformin hydrochloride with the specific release technical requirements said in the present invention can produce the best therapeutic effect and the lowest adverse reaction. The said preparation of the present invention has the following drug release characteristics: the dissolution rate of metformin hydrochloride in a pH 4.5 medium is not higher than 15% within 2 hours, and the dissolution rate of metformin hydrochloride in a pH 6.8 medium is not less than 85% within 20 minutes. More preferably the dissolution rate of metformin hydrochloride in a pH 4.5 medium is not higher than 7.5 % within 2 hours, and the dissolution rate in a pH 6.8 medium is more than 85% within 15 minutes. For enteric-coated tablets, achieving rapid release in a pH 6.8 medium requires the selection of enteric materials with a lower onset dissolving point or a lower weight gain in coating, but enteric materials with a lower onset dissolving point or a lower weight gain in coating will easily lead to increased release in a pH 4.5-5.0 medium. The present invention has obtained that by controlling the disintegration time of tablet core, the enteric coating material with the onset dissolving point and the weight gain in coating, especially controlling enteric coating material with the onset dissolving point and the weight gain in coating, the prepared metformin hydrochloride enteric-coated tablets can meet the above-mentioned drug release technical requirements. The metformin hydrochloride enteric-coated tablets said in the present invention improve the gastrointestinal tolerance of metformin hydrochloride without reducing or even improving the hypoglycemic efficacy and reduce the adverse reactions caused by the drug, which are significantly different from the IR (Immediate Release) or enteric-coated preparations of metformin hydrochloride with non-above release characteristics. Further studies on formulation/process and in vitro and in vivo correlation showed that the metformin hydrochloride enteric-coated tablets using enteric coating materials with different onset dissolving points can meet the above-mentioned technical requirements for drug release under the condition of controlling appropriate weight gain in coating and disintegration time of tablet core, and are bioequivalent to the metformin hydrochloride enteric-coated tablets of the preferred Example (E1).

### BRIEF DESCRIPTION OF THE DRAWINGS

Figure 1: HbA1c reduction during 12 weeks of treatment with E1, D2, D5 and Glucophage (R)
Figure 2: FPG change of E1, D2, D5 and Glucophage (R)
Figure 3: AE of E1, D2, D5 and Glucophage (R)
Figure 4: Dissolution curves (pH 6.8) of E1, D2, and D5
Figure 5: Plasma concentration - time curves of E1, D2, D5 and Glucophage (R)
Figure 6: Dissolution curves (pH 6.8) of E1, E10, E13 and D4
Figure 7: Plasma concentration - time curves of E1, E10, E13 and D4
Figure 8: Dissolution curves (pH 6.8) of E1, E4 and D1
Figure 9: Plasma concentration - time curves of E1, E4 and D1
Figure 10: Distribution and flow chart of subjects

### DETAILED DESCRIPTION OF THE EXAMPLES

The following specific examples are only used to explain or illustrate the content of the present invention. Obviously, the implementation mode of the present invention is not limited to the specific examples listed, so they should not be understood as constituting any limitation on the protection scope of the claims of this patent application.

The tablet cores, coating materials and weight gain in coating of the metformin hydrochloride enteric-coated tablets in the examples of the present invention and in the comparative examples are shown in Table 1 below.

**Table 1 Overview of tablet cores and coatings in Examples and Comparative Examples**

| Examples | Coating material | Weight gain in coating % (mg/cm² ) | Tablet core | Notes |
|---|---|---|---|---|
| Example 1 (E1) | 5.5-B1 | 4.5 (7.2) | Rapid disintegration - **VV | +++, disintegration time 2-4min |
| Example 2 (E2) | 5.5-B1 | 3.5 (5.6) | Same as E1 | +++ |
| Example 3 (E3) | 5.5-B1 | 7.0 (11.2) | Same as E1 | ++ |
| Example 4 (E4) | 5.5-B1 | 4.5 (7.2) | Slow disintegration -VV2 | ++, disintegration time 6-8min |
| Example 5 (E5) | 5.5-B2 | 5.5 (8.8) | Same as E1 | +++ |
| Example 6 (E6) | 5.5-B 3 | 4.5 (7.2) | Same as E1 | +++ |
| Example 7 (E7) | 5.5-B 4 | 5.8 (9.3) | Same as E1 | +++ |
| Example 8 (E8) | 5.5 - A1+C1 | 5.0 (8.0) | Same as E1 | +++ |
| Example 9 (E9) | 5.5-B1 | 5.5 (8.8) | Same as E1 | +++ |
| Example 10 (E10) | 5.0-A1 | 8.0 (12.8) | Same as E1-VV1 | ++ |
| Example 11 (E11) | 5.0-A1 | 6.0 (9.6) | Same as E1 | +++ |
| Example 12 (E12) | 5.0-A1 | 4.0 (6.4) | Same as E1 | +++, pH 4.5 - 2h dissolution 11.9 % |
| Example 13 (E13) | 6.0-C1 | 3.5 (5.6) | Same as E1-VV1 | +++ |
| Example 14 (E14) | 6.0-C1 | 3.0 (4.8) | Same as E1 | +++, pH 4.5 - 2h dissolution 14.6 % |
| Example 15 (E15) | 6.0-C1 | 2.5 (4.0) | Same as E1 | +++, Coating isolation layer |
| Example 16 (E16) | 6.0-C1 | 4.5 (7.2) | Same as E1 | ++ |
| Example 17 (E17) | 6.0-C1 | 5.5 (8.8) | Same as E1 | ++ |
| Example 18 (E18) | 6.0-C2 | 3.5 (5.6) | Same as E1 | +++ |
| Example 19 (E19) | 6.0-C3 | 3.5 (5.6) | Same as E1 | +++ |
| Example 20 (E20) | 5.5-B1 | 3.0 (4.8) | Same as E1 | +++, Coating isolation layer |
| Example 21 (E21) | 5.0-A1 | 3.5 (5.6) | Same as E1 | +++, Coating isolation layer |
| Comparative Example 1 (D1) | 5.5-B1 | 4.5 (7.2) | Slow disintegration -VV2 | Disintegration time 12-15min |
| Comparative Example 2 (D2) | 5.0-A1 | 2.5 (4.0) | Same as E1-** | +++, pH 4.5 - 2h dissolution 21.6 % |
| Comparative Example 3 (D3) | 6.0-C1 | 2.0 (3.2) | Same as E1 | +++, pH 4.5 - 2h dissolution 27.8% |
| Comparative Example 4 (D4) | 6.0-C1 | 6.5 (10.4) | Same as E1 -VV1 | pH 6.8 - 15 minutes dissolution 25%, 20 minutes dissolution 73% |
| Comparative Example 5 (D5) | 6.5 -D1 | 3.8 (6.1) | Same as D1 -** | Coating isolation layer, pH 6.8 - 20 minutes dissolution 39.5% |
| Comparative Example 6 (D6) | 5.5-B1 | 2.5 (4.0) | Same as E1 | +++, pH 4.5 - 2h dissolution 21.9% |
| Comparative Example 7 (D7) | 5.5-B1 | 9.0 (14.4) | Same as E1 | |
| Comparative Example 8 (D8) | 5.0-A1 | 10 (16.0) | Same as E1 | |

| | | | | |
|---|---|---|---|---|
| Notes: A: The onset dissolving point is pH 5.0. B: The onset dissolving point is pH 5.5. C: The onset dissolving point is pH 6.0. D: The onset dissolving point is pH 6.5. A1: Hydroxypropyl methylcellulose phthalate (HP-50) B1: Methacrylic acid ethyl acrylate copolymer (L100-55) B2: Methacrylic acid ethyl acrylate copolymer (L30D-55) B3: Hydroxypropyl methylcellulose phthalate (HP-55 or HP-55S) B4: Hydroxypropyl methylcellulose acetate succinate (HPMCAS-L) C1: methacrylic acid methyl methacrylate copolymer (L100; polyacrylic resin II) C2: Hydroxypropyl methylcellulose acetate succinate (HPMCAS-M) C3: Cellulose acetate phthalate (CAP; Cellulose acetate) D1: Eudragit FS + Eudragit L (pH 6.5) ++: Dissolution rate of more than 85% in 20 minutes in pH 6.8 medium +++: Dissolution rate of more than 85% in 15 minutes in pH 6.8 medium **: Clinical trial and pharmacokinetic trial examples VV1: in vitro and in vivo correlation trial (1) examples VV2: in vitro and in vivo correlation trial (2) examples Conversion method between weight gain in coating "%" and "mg/cm²": mg/cm² =X * weight of tablet core /surface area ("X" is the percentage of weight gain in coating, for example, "X=5%"=means the weight gain in coating is 5%). For the surface area of the preparation, please refer to the methods in the literature such as "Eutrogenic Manual - Surface Area Calculation". | | | | |

### Example 1 (E1): Metformin hydrochloride enteric-coated tablets

Composition of tablet core:

| **Ingredients** | **Dosage ( mg )** |
|---|---|
| Metformin Hydrochloride | 500.00 |
| Corn starch | 16.54 |
| Mannitol | 20.00 |
| Sodium starch glycolate | 16.68 |
| Magnesium Stearate (Added externally) | 2.78 |
| Purified water | Moderate |
| Tablet weight | 556 |

Preparation method : Metformin hydrochloride is crushed and passed through an 80 -mesh sieve. The crushed API and the internal excipients are placed in a wet granulator for mixing for 5 minutes, and then purified water is added for wet granulation. The wet granules are granulated using a granulator (with a 5×5mm square hole screen installed) and then put into a fluid bed drier for drying. The moisture content of the dry granules is controlled within the range of 1.0 to 4.0%. After the dry granules are granulated (the granulator is equipped with a 1.5mm round hole screen), magnesium stearate is added and mixed in a blender for 5 minutes. A 12mm round punch is used for tableting, and the hardness of the plain tablets is controlled at 8 to 11 kg.

According to the disintegration time test method in General Chapter 0921 of Part IV of the 2020 edition of the "Chinese Pharmacopoeia", it was measured that the plain tablets disintegrated completely within 2 to 4 minutes.

Composition of enteric coating solution:

| **Ingredients** | **Dosage ( g )** |
|---|---|
| Methacrylic acid ethyl acrylate copolymer ( L100-55 ) | 6.00 |
| Triethyl citrate | 0.60 |
| Talc | 1.50 |
| Ethanol (90%) | 91.90 |

Enteric layer coating: Add methacrylic acid ethyl acrylate copolymer (L100-55) to ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 4.5% (7.2mg/cm²).

### Example 2 (E2): Metformin hydrochloride enteric-coated tablets

The tablet core was prepared according to the tablet core composition and preparation method of Example 1. The enteric coating was prepared according to the enteric coating solution composition and process of Example 1, and the weight gain in coating was 3.5 % ( 5.6 mg/cm² ) .

### Example 3 (E3): Metformin hydrochloride enteric-coated tablets

Tablet cores were prepared according to the tablet core composition and preparation method of Example 1. The enteric coating was prepared according to the enteric coating solution composition and process of Example 1 with the weight gain in coating being 7.0 % (11.2 mg/cm²).

### Example 4 (E4): Metformin hydrochloride enteric-coated tablets

The total mixed granules were obtained according to the core composition and preparation method of Example 1. The hardness of the plain tablets was controlled at 15-18 kg during tableting (a small amount of binder was added if necessary). According to the disintegration time test method in General Chapter 0921 of Part IV of the 2020 edition of the "Chinese Pharmacopoeia", it was measured that the plain tablets disintegrated completely within 6-8 minutes. The enteric coating solution composition, process and weight gain in coating were the same as those in Example 1.

### Example 5 (E5): Metformin hydrochloride enteric-coated tablets

The tablet core was prepared according to the tablet core composition and preparation method of Example 1 .

Composition of enteric coating solution:

| **Ingredients** | **Dosage ( g )** |
|---|---|
| Methacrylic acid ethyl acrylate copolymer ( L30D-55 aqueous dispersion) | 33.33 (content 30%) |
| Polyethylene glycol 6000 | 1.00 |
| Talc | 2.50 |
| Purified water | 63.17 |

Enteric layer coating: Add polyethylene glycol 6000 and talc to purified water, use a high shear homogenizer to fully homogenize for 5 to 10 minutes, then slowly pour the above liquid into the L30D-55 aqueous dispersion, slowly stir to disperse evenly, and pass through an 80- mesh sieve before coating. Use a coating machine to coat the enteric layer, dry it after coating to age the enteric layer, and the enteric layer weight gain in coating is 5.5% (8.8 mg/cm²).

### Example 6 (E6): Metformin hydrochloride enteric-coated tablets

The tablet core was prepared according to the tablet core composition and preparation method of Example 1 .

Composition of enteric coating solution:

| **Ingredients** | **Dosage ( g )** |
|---|---|
| Hydroxypropyl methylcellulose phthalate ( HP-55 ) | 8.00 |
| Triethyl citrate | 0.40 |
| Talc | 2.40 |
| Ethanol (80%) | 89.20 |

Enteric layer coating: Use ethanol (95%) and purified water to make ethanol (80%), add hydroxypropyl methylcellulose phthalate into ethanol (80%), stir to completely dissolve, then add triethyl citrate and talc in turn and stir to disperse evenly. Use a coating machine to coat the enteric layer, dry after coating to remove residual solvent, and the weight gain of the enteric layer coating is 4.5% (7.2mg/cm²).

### Example 7 (E7): Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Hydroxypropyl methylcellulose acetate succinate ( HPMCAS-L ) | 7.00 |
| Triethyl citrate | 1.40 |
| Talc | 2.10 |
| Sodium Lauryl Sulfate | 0.21 |
| Purified water | 89.29 |

Enteric layer coating: Cool the purified water to below 25°C, add triethyl citrate and sodium lauryl sulfate under stirring, and stir until completely dissolved. Then gradually add hydroxypropyl methylcellulose acetate succinate (micro powder type) and talc, stir to disperse evenly, pass through a 100- mesh sieve before coating, and use a coating machine to coat the enteric layer. The coating liquid needs to be cooled during the coating process, preferably kept below 15°C. After coating, dry the enteric layer for aging treatment. The weight gain of the enteric layer coating is 5.8% (9.3mg/cm²).

### Example 8 (E8): Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Methacrylic acid methyl methacrylate copolymer (Eudragit L100 ) | 3.50 |
| Hydroxypropyl methylcellulose phthalate ( HP-50 ) | 3.50 |
| Triethyl citrate | 0.60 |
| Talc | 1.75 |
| Ethanol (90%) | 90.65 |

Enteric layer coating: Add methacrylic acid methyl methacrylate copolymer and hydroxypropyl methylcellulose phthalate into ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 5.0% (8.0 mg/cm²).

### Example 9 (E9): Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Methacrylic acid ethyl acrylate copolymer ( Eudragit L100-55 ) | 6.00 |
| Polyethylene glycol 6000 | 0.60 |
| Talc | 4.50 |
| Ethanol (90%) | 88.90 |

Enteric layer coating: Add methacrylic acid ethyl acrylate copolymer to ethanol (90%) and stir to completely dissolve. Then add polyethylene glycol 6000 and talc in turn and stir to disperse evenly. Use a coating machine to coat the enteric layer. After coating, dry to remove residual solvent. The enteric layer weight gain in coating is 5.5% (8.8 mg/cm² ) .

### Example 10 (E10): Metformin hydrochloride enteric-coated tablets

The tablet core was prepared according to the tablet core composition and preparation method of Example 1 .

Composition of enteric coating solution:

| **Ingredients** | **Dosage ( g )** |
|---|---|
| Hydroxypropyl methylcellulose phthalate ( HP-50 ) | 8.00 |
| Triethyl citrate | 0.40 |
| Talc | 2.40 |
| Ethanol (80%) | 89.20 |

Enteric layer coating: Add Hydroxypropyl Methylcellulose Phthalate to ethanol (80%), stir until completely dissolved, then add triethyl citrate and talc in turn and stir until evenly dispersed. Use a coating machine to coat the enteric layer, dry after coating to remove residual solvent, and the weight gain of the enteric layer coating is 8.0 % (12.8 mg/cm² ) .

### Example 11 (E11): Metformin hydrochloride enteric-coated tablets

Tablet cores were prepared according to the tablet core composition and preparation method of Example 1, and enteric coating was prepared according to the composition of the enteric coating solution and process of Example 10, with the weight gain in coating being 6.0 % ( 9.6 mg/cm² ) .

### Example 12 (E12): Metformin hydrochloride enteric-coated tablets

The tablet core was prepared according to the tablet core composition and preparation method of Example 1, and the enteric coating was prepared according to the composition of enteric coating solution and process of Example 10, and the weight gain in coating was 4.0 % ( 6.4 mg/cm² ) .

### Example 13 (E13): Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Methacrylic acid methyl methacrylate copolymer ( Eudragit L100 ) | 6.00 |
| Triethyl citrate | 0.90 |
| Talc | 1.50 |
| Ethanol (90%) | 91.60 |

Enteric layer coating: Add methacrylic acid methyl methacrylate copolymer into ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 3.5% (5.6 mg/cm²).

### Example 14 (E14) : Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Methacrylic acid methyl methacrylate copolymer (Eudragit L100 ) | 6.00 |
| Triethyl citrate | 0.90 |
| Talc | 0.90 |
| Ethanol (90%) | 92.20 |

Enteric layer coating: Add methacrylic acid methyl methacrylate copolymer to ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 3.0 % (4.8 mg/cm²).

### Example 15 (E15): Metformin hydrochloride enteric-coated tablets

The tablet core was prepared according to the tablet core composition and preparation method of Example 1.

Composition of isolation layer coating solution:

| **Ingredients** | **Dosage ( g )** |
|---|---|
| Gastric soluble film coating premix (Opadry 85F ) | 15.0 |
| Purified water | 85.0 |

Isolation layer coating: Add the gastric soluble film coating premix into purified water, stir to disperse evenly, and sieve through 80 mesh before coating. Use a coating machine to coat the isolation layer, dry it after coating to remove the residual moisture, and the weight gain in coating is 2.0%.

Composition of enteric coating solution:

| **Ingredients** | **Dosage ( g )** |
|---|---|
| Methacrylic acid methyl methacrylate copolymer (Eudragit L100 ) | 6.00 |
| Triethyl citrate | 0.6 |
| Talc | 1.50 |
| Ethanol (90%) | 91.90 |

Enteric layer coating: Add methacrylic acid methyl methacrylate copolymer into ethanol(90%) and stir to completely dissolve. Then add triethyl citrate and talc in turn and stir to disperse evenly. Use a coating machine to coat the enteric layer. After coating, dry to remove residual solvent. The weight gain of the enteric layer coating is 2.5% (4.0 mg/cm²).

### Example 16 (E16): Metformin hydrochloride enteric-coated tablets

The tablet core was prepared according to the tablet core composition and preparation method of Example 1 .

Composition of enteric coating solution:

| **Ingredients** | **Dosage ( g )** |
|---|---|
| Methacrylic acid methyl methacrylate copolymer (Eudragit L100 ) | 6.00 |
| Triethyl citrate | 0.6 |
| Talc | 1.50 |
| Ethanol (90%) | 91.90 |

Enteric layer coating: Add methacrylic acid methyl methacrylate copolymer into ethanol (90%) and stir to completely dissolve. Then add triethyl citrate and talc in turn and stir to disperse evenly. Use a coating machine to coat the enteric layer. After coating, dry to remove residual solvent. The weight gain of the enteric layer coating is 4.5 % (7.2 mg/cm²).

### Example 17 (E17): Metformin hydrochloride enteric-coated tablets

The tablet core was prepared according to the tablet core composition and preparation method of Example 1 .

The composition of enteric layer coating solution and process were the same as those in Examples 1-6, and the enteric layer weight gain in coating was 5.5 % ( 8.8 mg/cm² ) .

### Example 18 (E18): Metformin hydrochloride enteric-coated tablets

The tablet core was prepared according to the tablet core composition and preparation method of Example 1 .

Composition of enteric coating solution:

| **Ingredients** | **Dosage ( g )** |
|---|---|
| Hydroxypropyl methylcellulose acetate succinate (HPMCAS-M) | 6.00 |
| Ethanol (80%) | 94.00 |

Enteric layer coating: Use ethanol (95%) and purified water to make ethanol (80%), add hydroxypropyl methylcellulose acetate succinate into ethanol (80%), stir to completely dissolve. Use a coating machine to coat the enteric layer, dry after coating to remove residual solvent, and the enteric layer weight gain in coating is 3.5% (5.6mg/cm²).

### Example 19 (E19): Metformin hydrochloride enteric-coated tablets

The tablet core was prepared according to the tablet core composition and preparation method of Example 1.

Composition of enteric coating solution:

| **Ingredients** | **Dosage ( g )** |
|---|---|
| Cellulose acetate ( CAP ) | 7.50 |
| Triacetin | 2.50 |
| Acetone | 90.00 |

Enteric layer coating: Add cellulose acetate and triacetin to acetone, stir until completely dissolved, and sieve through a 100- mesh sieve before use. Use a coating machine to coat the enteric layer, and dry to remove residual solvent after coating. The weight gain of the enteric layer coating is 3.5% (5.6 mg/cm²).

### Example 20 (E20): Metformin hydrochloride enteric -coated tablets

The tablet core was prepared according to the tablet core composition and preparation method of Example 1. The isolation coating was prepared according to the coating solution composition and process of Example 15, and the isolation coating weight was increased by 2.0%. The enteric coating was prepared according to the enteric layer coating solution composition and process of Example 1, and the enteric layer coating weight was increased by 3.0 % (4.8 mg/cm²).

### Example 21 (E21): Metformin hydrochloride enteric -coated tablets

The tablet core was prepared according to the tablet core composition and preparation method of Example 1. The isolation coating was prepared according to the coating solution composition and process of Example 15, and the isolation coating weight was increased by 2.0%. The enteric coating was prepared according to the enteric layer coating solution composition and process of Example 10, and the enteric layer coating weight was increased by 3.5 % (5.6 mg/cm²).

### Comparative Example 1 (D1): Metformin hydrochloride enteric-coated tablets

The composition of the tablet core is based on Glucophage (Metformin Hydrochloride Tablets), specifically:

| **Ingredients** | **Dosage ( mg )** |
|---|---|
| Metformin Hydrochloride | 500.00 |
| Povidone K30 | 20.00 |

| Magnesium Stearate (Added externally) | 4.00 |
|---|---|
| Purified water | Moderate |
| Tablet weight | 524 |

Preparation method : dissolve povidone K30 in purified water to prepare a binder for use, grind metformin hydrochloride and pass it through a 100 -mesh sieve, place the ground raw materials in a fluid bed granulating drier, and then spray the binder for one-step granulation, control the moisture content of the dry granules within the range of 1.0 to 4.0%, granulate the dry granules (the granulator is equipped with a 1.5 mm round hole screen), add magnesium stearate, mix in a blender for 5 minutes, and then use a 12 mm round punch for tableting, and the hardness of the plain tablets is controlled within the range of 11 to 15 kg .

According to the disintegration time test method in General Chapter 0921 of Part IV of the 2020 edition of the "Chinese Pharmacopoeia", it was measured that the plain tablets disintegrated completely within 12 to 15 minutes .

The composition of the enteric layer coating solution, process and weight gain in coating are the same as those in Example 1.

### Comparative Example 2 (D2): Metformin Hydrochloride Enteric-coated Tablets

Tablet cores were prepared according to the tablet core composition and preparation method of Example 1, and enteric coating was performed according to the composition of the enteric layer coating solution and process of Example 10, with the weight gain in coating being 2.5% (4.0 mg/cm² ) .

### Comparative Example 3 (D3): Metformin Hydrochloride Enteric-coated Tablets

Tablet cores were prepared according to the tablet core composition and preparation method of Example 1, and enteric coating was performed according to the enteric layer coating solution composition and process of Example 13, with the weight gain in coating being 2.0% (3.2 mg/cm² ) .

### Comparative Example 4 (D4): Metformin Hydrochloride Enteric-coated Tablets

Tablet cores were prepared according to the tablet core composition and preparation method of Example 1, and enteric coating was performed according to the enteric layer coating solution composition and process of Example 16, with the weight gain in coating being 6.5 % ( 10.4 mg/cm² ) .

### Comparative Example 5 (D5): Metformin Hydrochloride Enteric-coated Tablets

The tablet core is based on Glucophage. The enteric coating material is Eudragit FS30D and Eudragit L30D-55 in a ratio of 4:6 and its onset dissolving point is close to pH value of 6.5. As follows:
The tablet core was prepared according to the tablet core composition and preparation method of Comparative Example 1.

Composition of isolation layer coating solution:

| **Ingredients** | **Dosage ( g )** |
|---|---|
| Gastric soluble film coating premix (Opadry 85F ) | 15.0 |
| Purified water | 85.0 |

Separation layer coating: Add the gastric soluble film coating premix into purified water, stir to disperse evenly, and sieve through 80 mesh before coating. Use a coating machine to coat the isolation layer, dry it after coating to remove the residual moisture, and the weight gain in coating is 1.1%.

Composition of enteric coating solution:

| **Ingredients** | **Dosage ( g )** |
|---|---|
| Methacrylic acid, methyl acrylate and methyl methacrylate copolymer ( Eudragit FS30D ) | 13.33 |
| Methacrylic acid ethyl acrylate copolymer (Eudragit L30D-55 aqueous dispersion) | 20.00 |
| Triethyl citrate | 1.0 |
| Talc | 2.50 |
| Purified water | 63.17 |

Enteric layer coating: Add triethyl citrate and talc to purified water, use a high shear homogenizer to fully homogenize for 5 to 10 minutes, then slowly pour the above liquid into the Eudragit FS30D and L30D-55 aqueous dispersion, slowly stir to disperse evenly, and pass through an 80- mesh sieve before coating. Use a coating machine to coat the enteric layer, dry it after coating to age the enteric layer, and the enteric layer weight gain in coating is 3.8% (6.1mg/cm²).

### Comparative Example 6 (D6): Metformin Hydrochloride Enteric-coated Tablets

Tablet cores were prepared according to the tablet core composition and preparation method of Example 1, and enteric coating was performed according to the composition of the enteric layer coating solution and process of Example 1, with the weight gain in coating being 2.5 % ( 4.0 mg/cm² ) .

### Comparative Example 7 (D7): Metformin Hydrochloride Enteric-coated Tablets

Tablet cores were prepared according to the tablet core composition and preparation method of Example 1, and enteric coating was performed according to the composition of the enteric layer coating solution and process of Example 1, and the weight gain in coating was 9.0 % ( 14.4 mg/cm² ) .

### Comparative Example 8 (D8): Metformin Hydrochloride Enteric-coated Tablets

The tablet core was prepared according to the tablet core composition and preparation method of Example 1. Enteric coating was performed according to the composition of the enteric layer coating solution and process of Example 10, and the weight gain in coating was 10.0 % (16.0 mg/cm²).

### Methods of dissolution test and results

Refer to the dissolution test conditions of metformin hydrochloride enteric-coated tablets in the 2020 edition of the "Chinese Pharmacopoeia" and determine it according to the dissolution and release determination method (General Chapter 0931 Method 1).

### Dissolution in acid:

Dissolution conditions: Use 900 ml of 0.1 mol/L hydrochloric acid solution as the dissolution medium, rotate at 100 rpm(revolutions per minute), follow the prescribed procedures, and take samples after 2 hours.

Test solution: Take 20 ml of the dissolution, filter, discard 10 ml of the initial filtrate, and take the subsequent filtrate.

Reference solution: Take an appropriate amount of metformin hydrochloride reference substance, weigh it accurately, dissolve it in 0.1 mol/L hydrochloric acid solution and quantitatively dilute it to make a solution containing about 28 µg per 1 ml.

Determination method: Take the test solution and the reference solution, measure the absorbance at a wavelength of 233nm respectively, and calculate the dissolution amount.

Limit: Not more than 10% of the labeled amount .

**Dissolution in pH 4.5 buffer:** Use 900 ml of pH 4.5 acetate buffer as the dissolution medium, Basket - rotating method, rotation speed of 100 rpm, follow the prescribed procedures, and take samples after 2 hours. The test solution, the reference solution and the determination method are the same as determined in the pH 6.0 and pH 6.8 medium.

**Dissolution in pH 6.0 and pH 6.8 buffer:** Take the rotating basket after 2 hours under the acid-resistant item, and immediately immerse it in 900ml of buffer preheated to 37°C±0.5°C. Keep the speed unchanged and continue to follow the prescribed procedures. Sampling is carried out after 5, 10, 15, 20, 30, 45 and 60 minutes.

Test solution: Take 20 ml of the dissolution, filter, discard 10 ml of the primary filtrate, accurately measure an appropriate amount of the subsequent filtrate, and quantitatively dilute with water to make a solution containing approximately 5 µg of metformin hydrochloride per 1 ml.

Reference solution: Take an appropriate amount of metformin hydrochloride reference substance, weigh accurately, dissolve in water and quantitatively dilute to make a solution containing about 5µg per 1ml.

Determination method: Take the test solution and the reference solution, measure the absorbance at a wavelength of 233nm respectively, and calculate the dissolution amount.

### Preparation of dissolution medium:

pH 4.5 medium (acetate buffer): weigh 1.80g of anhydrous sodium acetate (or 2.99g of sodium acetate trihydrate), add 1.596ml of glacial acetic acid to 1L of degassed purified water, stir until completely dissolved, and adjust the pH value to 4.50 with glacial acetic acid.

pH 6.0 medium (phosphate buffer): Take 6.80g of potassium dihydrogen phosphate and 0.224g of sodium hydroxide and dissolve them in 1000ml of purified water. If necessary, adjust the pH value to 6.0 ± 0.05 with 2mol/L hydrochloric acid solution or 2mol /L sodium hydroxide solution.

pH 6.8 medium (phosphate buffer): Take 0.1mol/L hydrochloric acid solution and 0.2mol/L sodium phosphate solution, mix them evenly at a ratio of 3 : 1, and adjust the pH value to 6.8±0.05 with 2mol/L hydrochloric acid solution or 2mol/L sodium hydroxide solution if necessary.

The dissolution test results of the metformin hydrochloride enteric-coated tablets of the examples and the comparative examples: Results of the acid resistance test were suitable. The dissolution results in the acid-resistant and pH 4.5 medium are shown in Table 2 (the pH 1.0 value is the average value of the acid-resistant data in the pH 6.0 and pH 6.8 medium). The dissolution results in the pH 6.0 and pH 6.8 medium are shown in Table 3 and Table 4 ( the dissolution at 0 in the chart is the acid-resistant 2h test value; 6 preparation units are used for each dissolution curve measurement ) .

**Table 2 Dissolution of the examples and comparative examples in pH 1.0 and pH 4.5 medium for 2 hours (%)**

| **Example** | **pH 1.0** | **pH4.5** | **Example** | **pH 1.0** | **pH4.5** |
|---|---|---|---|---|---|
| Example 1 | 0.3 | 3.9 | Example 12 | 2.3 | 11.9 |
| Example 2 | 3.2 | 12.6 | Example 13 | 2. 7 | 6.9 |
| Example 3 | 0.0 | 0.0 | Example 14 | 4.5 | 14.6 |
| Example 4 | 0.2 | 2.9 | Example 15 | 2.9 | 11.2 |
| Example 5 | 0.3 | 4.2 | Example 16 | 0.1 | 1.7 |
| Example 6 | 0.3 | 2.3 | Example 17 | 0.3 | 0.0 |
| Example 7 | 0.2 | 1.8 | Example 18 | 3.1 | 8.7 |
| Example 8 | 0.7 | 4.2 | Example 19 | 1.8 | 5.2 |
| Example 9 | 0.4 | 2.7 | Example 20 | 2.3 | 6.7 |
| Example 10 | 0 | 0.8 | Example 21 | 2.6 | 11.1 |
| Example 11 | 0.6 | 3.0 | | | |
| Comparative Example 1 | 0.5 | 1.8 | Comparative Example 5 | 0 | 1.5 |
| Comparative Example 2 | 6.3 | 21.6 | Comparative Example 6 | 5.7 | 21.9 |
| Comparative Example 3 | 9.6 | 27.8 | Comparative Example 7 | 0.0 | 0.0 |
| Comparative Example 4 | 0 | 0 | Comparative Example 8 | 0.0 | 0.0 |

**Table 3 Dissolution of Examples and Comparative Examples in pH 6.0 Medium (%)**

| Time ( min ) | **0** | **5** | **10** | **15** | **20** | **30** | **45** | **60** |
|---|---|---|---|---|---|---|---|---|
| Example 1 | 0.5 | 0.5 | 1.2 | 9.3 | 35.0 | 93.5 | 97.1 | 98.6 |
| Example 2 | 3.1 | 3.0 | 15.0 | 71.6 | 95.0 | 95.6 | 96.2 | 95.9 |
| Example 3 | 0.0 | 0.0 | 0.5 | 1.2 | 5.2 | 40.7 | 93.1 | 98.8 |
| Example 4 | 0.3 | 0.6 | 1.1 | 8.3 | 22.2 | 65.8 | 95.9 | 99.5 |
| Example 5 | 0.2 | 1.0 | 5.3 | 18.0 | 48.0 | 93.1 | 98.6 | 99.1 |
| Example 6 | 0.2 | 0.6 | 1.6 | 16.3 | 45.6 | 92.7 | 98.9 | 100.1 |
| Example 7 | 0.3 | 2.0 | 3.9 | 16.4 | 55.3 | 96.5 | 98.2 | 99.1 |
| Example 8 | 0.5 | 1.2 | 1.5 | 18.6 | 51.3 | 92.8 | 97.7 | 98.0 |
| Example 9 | 0.5 | 1.0 | 2.2 | 11.0 | 38.6 | 91.6 | 97.8 | 99.1 |
| Example 10 | 0.0 | 0.1 | 2.5 | 38.1 | 85.5 | 96.0 | 100.2 | 101.2 |
| Example 11 | 0.7 | 0.1 | 16.7 | 72.5 | 95.3 | 98.5 | 98.9 | 100.2 |
| Example 12 | 2.2 | 8.7 | 72.8 | 91.1 | 96.2 | 98.2 | 98.1 | 97.4 |
| Example 13 | 2.5 | 0.1 | 0.6 | 1.0 | 2.3 | 5.0 | 13.5 | 32.7 |
| Example 14 | 4.8 | 0.2 | 0.3 | 0.3 | 1.5 | 6.7 | 26.9 | 51.4 |
| Example 15 | 4.7 | 0.1 | 0.1 | 0.2 | 0.3 | 3.6 | 13.8 | 41.7 |
| Example 16 | 0.1 | 0.1 | 0.0 | 0.2 | 0.1 | 0.2 | 1.2 | 15.6 |
| Example 17 | 0.1 | 0.2 | 0.1 | 0.3 | 0.3 | 0.3 | 1.5 | 11.0 |
| Example 18 | 2.9 | 0.2 | 0.5 | 1.3 | 2.8 | 6.4 | 16.0 | 37.6 |
| Example 19 | 1.9 | 0.2 | 0.1 | 0.3 | 0.5 | 2.0 | 11.5 | 25.0 |
| Example 20 | 2.6 | 2.7 | 10.3 | 63.1 | 98.0 | 98.3 | 98.8 | 98.5 |
| Example 21 | 2.0 | 3.5 | 65.6 | 87.3 | 97.5 | 99.0 | 98.8 | 99.1 |
| Comparative Example 1 | 0.4 | 0.5 | 0.9 | 2.6 | 11.3 | 38.5 | 79.3 | 95.8 |
| Comparative Example 2 | 7.2 | 16.2 | 79.3 | 93.1 | 94.3 | 93.9 | 94.0 | 94.1 |
| Comparative Example 3 | 8.9 | 1.0 | 3.1 | 6.3 | 13.3 | 32.6 | 51.0 | 81.6 |
| Comparative Example 4 | 0.0 | 0.1 | 0.0 | 0.0 | 0.1 | 0.2 | 1.8 | 3.1 |
| Comparative Example 5 | 0.0 | 0.0 | 0.1 | 0.2 | 0.2 | 0.3 | 0.4 | 0.5 |
| Comparative Example 6 | 5.6 | 8.7 | 23.9 | 88.2 | 94.6 | 94.0 | 94.8 | 94.5 |
| Comparative Example 7 | 0.0 | 0.0 | 0.0 | 0.2 | 1.6 | 26.8 | 78.0 | 99.9 |
| Comparative Example 8 | 0.0 | 0.0 | 0.0 | 19.5 | 71.0 | 94.6 | 99.0 | 99.5 |

**Table 4 Dissolution of Examples and Comparative Examples in pH 6.8 Medium (%)**

| Time ( min ) | 0 | 5 | 10 | 15 | 20 | 30 | 45 | 60 |
|---|---|---|---|---|---|---|---|---|
| Example 1 | 0.1 | 8.2 | 69.3 | 93.8 | 101.1 | 99.8 | 102.1 | 101.5 |
| Example 2 | 3.3 | 21.5 | 85.6 | 97.6 | 97.2 | 96.8 | 96.7 | 97.1 |
| Example 3 | 0.1 | 1.2 | 11.5 | 71.3 | 95.6 | 100.2 | 101.0 | 101.2 |
| Example 4 | 0.1 | 5.4 | 45.8 | 81.3 | 93.6 | 98.0 | 99.7 | 101.3 |
| Example 5 | 0.3 | 7.6 | 68.4 | 90.6 | 99.6 | 99.8 | 100.6 | 100.1 |
| Example 6 | 0.3 | 5.9 | 65.9 | 92.8 | 99.5 | 99.9 | 100.2 | 100.1 |
| Example 7 | 0.1 | 12.5 | 73.5 | 95.7 | 100.1 | 99.7 | 99.9 | 100.6 |
| Example 8 | 0.9 | 12.6 | 72.6 | 95.4 | 98.9 | 99.3 | 99.5 | 99.1 |
| Example 9 | 0.3 | 15.6 | 74.3 | 94.8 | 99.6 | 99.9 | 99.8 | 100.2 |
| Example 10 | 0.1 | 0.2 | 11.2 | 71.1 | 93.3 | 98 | 100.2 | 99.8 |
| Example 11 | 0.5 | 0.2 | 23.8 | 85.5 | 98.9 | 99.9 | 100.0 | 99.8 |
| Example 12 | 2.4 | 13.2 | 93.6 | 97.9 | 98.0 | 98.3 | 97.9 | 98.1 |
| Example 13 | 2.8 | 3.2 | 60.8 | 95.6 | 98.1 | 97.5 | 98.2 | 98.3 |
| Example 14 | 4.1 | 8.4 | 71.6 | 92.6 | 97.3 | 97.5 | 97.9 | 97.7 |
| Example 15 | 1.1 | 5.7 | 66.9 | 95.6 | 99.1 | 99.9 | 100.2 | 100.1 |
| Example 16 | 0.1 | 1.0 | 35.0 | 80.6 | 94.6 | 97.8 | 98.6 | 99.5 |
| Example 17 | 0.0 | 0.3 | 10.2 | 45.6 | 88.8 | 98.0 | 99.6 | 99.8 |
| Example 18 | 3.3 | 3.6 | 57.8 | 92.7 | 97.6 | 97.5 | 97.1 | 96.8 |
| Example 19 | 1.7 | 3.6 | 52.8 | 89.0 | 96.0 | 98.9 | 98.3 | 98.6 |
| Example 20 | 2.0 | 16.3 | 81.1 | 97.1 | 97.8 | 98.8 | 98.5 | 99.1 |
| Example 21 | 3.2 | 7.1 | 89.3 | 98.2 | 98.8 | 99.3 | 99.9 | 99.5 |
| Comparative Example 1 | 0.5 | 6.0 | 22.6 | 54.3 | 77.7 | 88.6 | 94.5 | 98.6 |
| Comparative Example 2 | 5.4 | 27.5 | 94.4 | 95.2 | 95.2 | 95.6 | 94.8 | 94.9 |
| Comparative Example 3 | 10.2 | 12.2 | 90.8 | 90.9 | 90.2 | 90.4 | 90.8 | 90.6 |
| Comparative Example 4 | 0.1 | 0.1 | 1.2 | 25.0 | 73.0 | 93.8 | 97.8 | 99.1 |
| Comparative Example 5 | 0.1 | 0.2 | 0.5 | 9.2 | 39.5 | 78.5 | 88.6 | 96.8 |
| Comparative Example 6 | 5.8 | 28.3 | 95.0 | 95.2 | 95.0 | 95.9 | 95.5 | 95.6 |
| Comparative Example 7 | 0.0 | 0.0 | 2.8 | 35.0 | 80.2 | 99.5 | 101.2 | 100.6 |
| Comparative Example 8 | 0.0 | 0.0 | 4.6 | 46.1 | 81.1 | 98.0 | 100.2 | 99.8 |

The enteric-coated metformin hydrochloride preparation disclosed in WO2014107617A requires that less than 15%, 10% or 5% of the biguanide compound be released in a hysteresis period of two hours at acidic pH and at least ten minutes or fifteen minutes at pH 6.8, and at least 60% of the biguanide compound be released within 60 minutes at pH 6.8 after the hysteresis period, and at least 90% of the biguanide compound be released within 90 to 120 minutes at pH 6.8. It can be seen that the dissolution of Comparative Example 5 meets the requirements of the enteric-coated metformin hydrochloride preparation described in the said patent document WO2014107617A.

### Clinical trials and results

Treatment-naive patients who were intolerant to ordinary immediate-release metformin hydrochloride tablets ( IR preparation) were randomly to four groups, administered metformin hydrochloride enteric-coated tablets of Comparative Example 2 (D2), Example 1 (E1) and Comparative Example 5 (D5), and ordinary immediate-release metformin hydrochloride tablets (reference preparation R, trade name Glucophage^{®}, commercially available) respectively, effects and adverse reactions were evaluated for metformin IR and DR with different release characteristics.

**Inclusion criteria:** Subjects who meet all of the following criteria can be included in this trial:
(1) Age: 28 to 78 years old, male or female;
(2) Diagnosed with type 2 diabetes according to the WHO (1999) diabetes diagnostic criteria ;
(3) Patients who have not taken other hypoglycemic drugs and are intolerant to ordinary immediate-release metformin hydrochloride tablets, including intolerance to gastrointestinal reactions such as diarrhea, nausea, vomiting, bloating, indigestion and abdominal discomfort, and adverse reactions of the nervous system such as dizziness and headache, and patients who have various adverse reactions that may be related to metformin IR. Patients should have been taking the drug for less than 2 weeks and are willing to take an alternative drug.
(4) Glycated hemoglobin ( HbA_{1c}) ≥7.0% and ≤11.0% at the screening visit;
(5) Fasting blood glucose (FPG) ≤15mmol/L (270mg/dL) at the screening visit ;
(6) Body mass index (BMI) ≥19 kg/m ² and ≤35 kg/m ²;

Dosage: Replace the metformin IR with the same dose of E1, D2, D5 and Glucophage. After replacement, increase the dose by 500 mg per week (take once in the morning and once in evening) until reaches 2000 mg/ day. For those who have already reached 2000 mg/ day, do not increase the dose and continue to take the replacement drug according to the original dose and method. For those who are still intolerant after changing the drug, the dose can be reduced to 1500 mg/ day or 1000 mg/ day.

During the treatment period, subjects who have other diseases that affects the evaluation ofefficacy and adverse events may withdraw from the trial. For subjects who withdraw due to adverse events, the adverse events will be recorded and the subjects would be included in the statistical analysis for safety review.

**Primary efficacy indicator:** Change in HbA_{1c} compared with baseline after 12 weeks of treatment.

### Secondary efficacy indicators:

(1) Changes in fasting blood glucose (FPG) compared with baseline at 4, 8, and 12 weeks of medication.
(2) Changes in body weight compared with baseline at 4, 8, and 12 weeks of medication.
(3) Changes in blood lipids (TC, TG, LDL-C, HDL-C) compared with baseline at 4, 8, and 12 weeks of medication.

Safety evaluation: adverse events were recorded during the study. Special attention was paid to the common gastrointestinal adverse reactions of metformin (including abdominal pain, diarrhea, constipation, abdominal distension, indigestion, etc.)

All medications (including combined medications) and adverse events of the patients were recorded in diary cards. The diary cards were collected at the next visit and medication compliance was calculated (by calculating the number of tablets used, generally requiring the actual number of tablets used to be > 80% of the theoretical amount). Patients with poor compliance were supervised. Subjects who took other hypoglycemic drugs were dropped from the study .

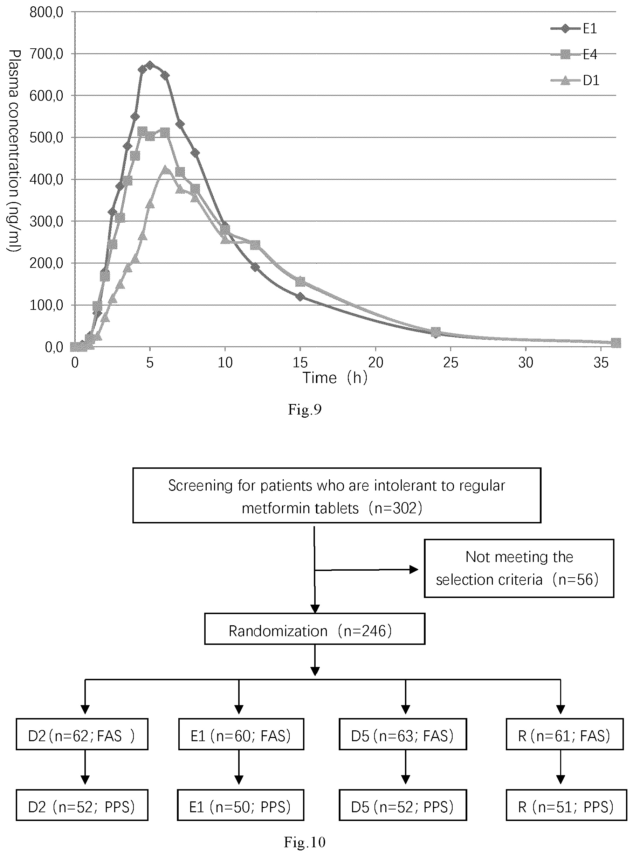

The trial process and subject distribution are shown in Table 5 and Figure 10. In Figure 10, FAS is the full analysis data set, which is all subjects who have taken the trial drug, and is used for safety / adverse event analysis. PPS is the per-protocol data set, which is all subjects who took the trial drug according to the protocol and obtained the 12 weeks'HbA1c, and it is used for efficacy analysis.

**Table 5 Trial process**

| **Stage** | **Screening period** | **Baseline period** | **Treatment period** | | |
|---|---|---|---|---|---|
| **Visit** | **Visit 1** | **Visit 2** | **Visit 3** | **Visit 4** | **Visit 5** |
| **Number of days** | **-D 14 -D0** | **D0** | **D28± 5** | **D56± 5** | **D84± 5** |
| Medical history / demographic data | X | | | | |
| Entry criteria | X | X | | | |
| Randomization | | X | | | |
| Distribute / collect medication and diary cards | | X | X | X | X |
| Adverse event records | | X | X | X | X |
| Treatment recommendations and medication instructions | X | X | X | X | X |
| Physical examination | X | X | X | X | X |
| Blood examination | X | X | X | X | X |
| Liver and kidney function examination | X | X * | X | X | X |
| HbA1c | X * | | | | X |
| FPG | X | X | X | X | X |
| Body weight | X | X | X | X | X |
| Blood lipids | X | X * | X | X | X |
| Uric acid | X | X * | X | X | X |

| | | | | | |
|---|---|---|---|---|---|
| **: No need to do this if done within 2 weeks; efficacy statistics are based on the value of the last examination before taking the medicine.* | | | | | |

### Results and discussion

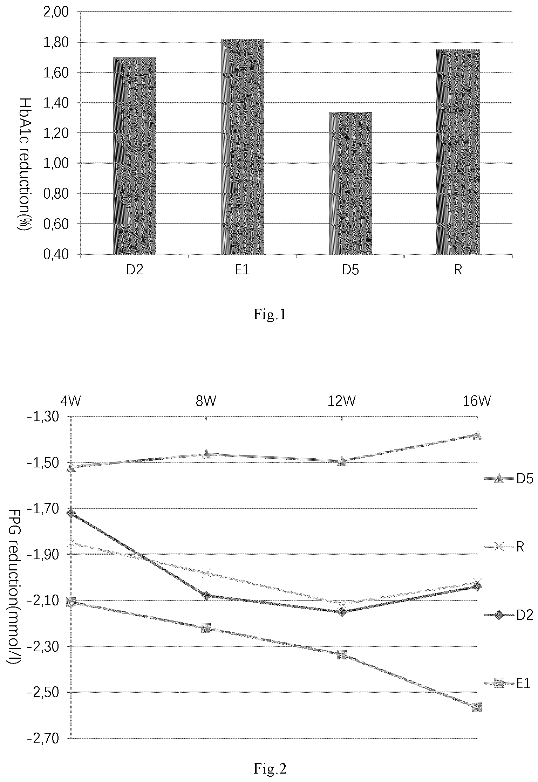

E1 had the best efficacy in terms of HbA1c, and fasting blood glucose also showed a similar trend. In addition, the reduction in fasting blood glucose showed an increasing trend as the duration of medication increased, indicating that E1 may have better long-term efficacy (see Figures 1-2 and Table 6. FPG at the 16^{th} week was an unscheduled test, with a small number of patients and a large time span, so no statistical analysis was performed); E1 was also superior to other preparations in terms of its effects on blood lipids, liver and kidney function. There was no significant difference between D2 and the control drug Glucophage in terms of hypoglycemic effect. D5 was significantly different from Glucophage ( P<0.05, and the magnitude exceeded the clinical non-inferiority margin of 0.3-0.4 ).

**Table 6: Efficacy**

| | | D2 **(n=52)** | E1 **(n=50)** | D5 **(n=52)** | R **(n=51)** |
|---|---|---|---|---|---|
| HbA1c, (%) | | | | | |
| | Baseline | 8.24 (1.03) | 8.37 (0.91) | 8.44 (1.26) | 8.21 (1.17) |
| | End of medication (3M) | 6.54 (0.58) | 6.55 (0.60) | 7.10 (1.05) | 6.45 (0.63) |
| | Reduction from baseline | 1.70 (0.93)** | 1.82 (1.08) ** | 1.34 (0.84) ** | 1.75 (1.19) ** |
| | p -value ( vs R) | 0.82 | 0.88 | 0.04 | |

| FPG, mmol/l | | | | | |
|---|---|---|---|---|---|
| | Baseline | 9.19 (1.69) | 9.45 (1.75) | 9.21 (2.13) | 9.01 (2.27) |
| | Reduction in 1M | 1.72 (1.23) ** | 2.11 (1.70) ** | 1.52 (1.42) ** | 1.85 (1.94) ** |
| | Reduction in 2M | 2.08 (1.31)** | 2.22 (1.90) ** | 1.46 (1.34) ** | 1.98 (2.03) ** |
| | Reduction in 3M | 2.15 (1.27) ** | 2.34 (1.98) ** | 1.49 (1.44) ** | 2.11 (2.22) ** |
| | p -value ( 3M vs R) | 0.82 | 0.55 | 0.10 | |

| Weight, kg | | | | | |
|---|---|---|---|---|---|
| | Baseline | 71.7±11.8 | 72.3±12.6 | 73.2±12.1 | 72.6±11.1 |
| | Reduction in 1M | 0.46±0.30* | 0.58±0.35** | 0.46±0.34* | 0.56±0.36** |
| | Reduction in 2M | 0.64±0.45** | 0.71±0.46** | 0.52±0.40** | 0.67±0.48** |
| | Reduction in 3M | 0.81±0.55** | 0.88±0.56** | 0.63±0.50** | 0.82±0.52** |
| | p -value ( 3M vs R) | 0.91 | 0.75 | 0.37 | |

| | | | | | |
|---|---|---|---|---|---|
| ** : P<0.05 compared with baseline ; ** : P<0.01 compared with baseline* E1, D2, and D5 were significantly superior to Glucophage in terms of upper gastrointestinal adverse events (AEs), such as nausea, vomiting, abdominal pain, abdominal discomfort, abdominal distension, and acid reflux, especially E1 and D5. For patients who experienced upper gastrointestinal adverse events while taking metformin hydrochloride tablets, the probability of experiencing upper gastrointestinal AEs after switching to E1 and D5 was only 33% of that before the medication change, while it was 93% for Glucophage. The improvement of D2 was not very obvious, and the probability of upper gastrointestinal AEs after the change of medication was 59% of that before the change of medication . E1 also had certain advantages in other AEs (see Table 7 and Figure 3) . | | | | | |

**Table 7: Adverse events**

| **Major adverse events** | D2 **(n=62)** | | | E1 **(n=60)** | | | D5 **(n=63)** | | | R **(n=61)** | | |
|---|---|---|---|---|---|---|---|---|---|---|---|---|
| | Screening | Trial | Ratio | Screening | Trial | Ratio | Screening | Trial | Ratio | Screening | Trial | Ratio |
| Nausea | 21 | 13 | 61.9% | 20 | 6 | 30.0%* | 26 | 8 | 30.8%* | 23 | 25 | 108.7% |
| Vomit | 6 | 3 | 50.0% | 8 | 3 | 37.5% | 11 | 3 | 27.3% | 8 | 6 | 75.0% |
| Stomach ache | 14 | 7 | 50.0% | 15 | 5 | 33.3% | 11 | 4 | 36.4% | 9 | 8 | 88.9% |
| Abdominal Discomfort | 5 | 4 | 80.0% | 8 | 3 | 37.5% | 10 | 3 | 30.0% | 11 | 8 | 72.7% |
| Abdominal Bloating | 18 | 10 | 55.6% | 13 | 4 | 30.8% | 17 | 6 | 35.3% | 17 | 15 | 88.2% |
| Acid reflux | 4 | 3 | 75.0% | 6 | 2 | 33.3% | 8 | 3 | 37.5% | 6 | 7 | 116.7% |
| Diarrhea | 31 | 27 | 87.1% | 32 | 23 | 71.9% | 35 | 45 | 128.6% | 26 | 28 | 107.7% |
| Constipation | 4 | 3 | 75.0% | 4 | 3 | 75.0% | 2 | 3 | 150.0% | 3 | 3 | 100.0% |
| Dizziness | 11 | 6 | 54.5% | 10 | 3 | 30.0% | 8 | 3 | 37.5% | 16 | 13 | 81.3% |
| Headache | 3 | 2 | 66.7% | 6 | 3 | 50.0% | 9 | 4 | 44.4% | 10 | 8 | 80.0% |
| Upper respiratory tract infection | 3 | 9 | 300.0% | 3 | 6 | 200.0% | 4 | 6 | 150.0% | 4 | 10 | 250.0% |
| **Main AE Total** | 120 | 87 | 72.5% | 125 | 61 | 48.8%* | 141 | 88 | 62.4%* | 133 | 131 | 98.5% |
| **AE Lead to withdrawal** | | 16 | 25.8% | | 7 | 11.7% | | 14 | 22.2% | | 21 | 34.4% |
| **Upper GI AE** | 68 | 40 | 58.8% | 70 | 23 | 32.9%* | 83 | 27 | 32.5%* | 74 | 69 | 93.2% |
| **Lower GI AE** | 35 | 30 | 85.7% | 36 | 26 | 72.2% | 37 | 48 | 129.7% | 29 | 31 | 106.9% |

| | | | | | | | | | | | | |
|---|---|---|---|---|---|---|---|---|---|---|---|---|
| *AE: adverse event* *Upper GI AEs (Upper gastrointestinal AEs) include: nausea, vomiting, abdominal pain, abdominal discomfort, bloating, acid reflux* *Lower GI AEs (Lower gastrointestinal AEs) include: diarrhea, constipation* *Ratio = adverse events during the trial period* / *adverse events during the screening period × 100% ;* ** : P<0.05 compared with R* | | | | | | | | | | | | |

The inventors conducted further analysis on the patients in group D2 and found that the occurrence of upper gastrointestinal AE in this group of patients was significantly related to the time of taking the medicine. patients who took the medicine before meals (30 minutes before meals) had upper gastrointestinal AE much lower than those who took the medicine after meals or during meals (E1 and D5 did not have such a phenomenon). This is obviously inconsistent with the adverse reaction characteristics of metformin reported in the literature. The literature shows that the adverse reactions of taking the medicine before meals are higher than those of taking it during meals and after meals. Therefore, the labeling of various countries require that metformin (ordinary preparations) be taken during meals (or immediately after meals) to reduce adverse gastrointestinal reactions.

After noticing the above phenomenon, the researchers compared adverse events of the drug before and after meals, the relationship between meal times and AEs as shown in Table 8.

**Table 8 : Comparison of adverse events in patients in group D2 after administration of medication before and after meals**

| Adverse Events | Before meal | | | During and after meals | | | D2 group total (n=62) | | |
|---|---|---|---|---|---|---|---|---|---|
| | Screening | Trial | Ratio | Screening | Trial | Ratio | Screening | Trial | Ratio |
| Nausea | 9 | 3 | 33% | 9 | 6 | 67% | 21 | 13 | 62% |
| Vomit | 2 | 0 | 0% | 3 | 2 | 67% | 6 | 3 | 50% |
| Stomach ache | 5 | 2 | 40% | 5 | 4 | 80% | 14 | 7 | 50% |
| Abdominal discomfort | 2 | 1 | 50% | 2 | 1 | 50% | 5 | 4 | 80% |
| Abdominal bloating | 6 | 2 | 33% | 8 | 5 | 63% | 18 | 10 | 56% |
| Acid reflux | 2 | 1 | 50% | 1 | 1 | 100% | 4 | 3 | 75% |
| Diarrhea | 11 | 7 | 64% | 9 | 6 | 67% | 31 | 27 | 87% |
| Dizziness | 4 | 2 | 50% | 5 | 3 | 60% | 11 | 6 | 55% |
| **Total upper GI AEs** | 26 | 9 | 35% | 28 | 19 | 68% | 68 | 40 | 59% |

| | | | | | | | | | |
|---|---|---|---|---|---|---|---|---|---|
| *upper GI(gastrointestinal) AE is the same as in the above table.* *Data that could not distinguish the correlation between medication* / *meal* /*AE were excluded because the diary card did not clearly record the time of medication and meal. Therefore, the number of people counted before meals + During meals and after meals was less than the total number of D2 groups.* | | | | | | | | | |

The inventor analyzed the reasons by comparing the formulation/process and dissolution of D2, and believed that it might be because D2 had a low onset dissolving point, a small weight gain in coating (thinner coating), a higher pH (up to 5 or higher) in the stomach and upper small intestine during and after meals, and faster gastric motility and delayed emptying after meals, coupled with food squeezing, which might cause the enteric coating to dissolve and rupture faster, thereby releasing the drug in the stomach . Before meals, the pH value in the stomach was lower, gastric motility was slow and gentle, and emptying was faster, so the enteric coating truly achieved the enteric effect, and the drug was released only after passing through the stomach, thus avoiding drug irritation to the upper digestive tract.

The inventors designed a dissolution test: simulated postprandial gastric juice (acetate buffer and milk were mixed in a ratio of 1:1, sodium hydroxide or hydrochloric acid was used to adjust the pH value to 5.0, acetate buffer contained 13.85g/L sodium chloride, 1.03g/L acetic acid, and 2.44g/L sodium acetate), paddle method, 75 rpm for D2 dissolution test, tablets began to break at 30 minutes, dissolution reached more than 75 % at 60 minutes, and metformin was completely dissolved at 90 minutes (detection method: high performance liquid chromatography, sample filtration and dilution (pre-column added if necessary), sulfonic acid cation exchange bonded silica gel as filler, 1.7% diammonium phosphate solution (phosphoric acid adjusted pH to 4.0) as mobile phase, detection wavelength of 233nm, column temperature of 30°C, flow rate of 1.3ml/min). The test results show that it is difficult to achieve the "enteric dissolution" effect when taking D2 after a meal.

The inventors re-performed the dissolution test of postprandial gastric juice with Example 12 (E12) and Example 15 (E15), and the test method was the same as above. The tablets of Example 12 (E12) began to break at about 60 minutes with a dissolution rate of about 20 %, and the dissolution rate reached more than 30 % at 90 minutes. The tablets of Example 15 (E15) began to swell and deform at about 60 minutes with a dissolution rate of about 10%, and the dissolution rate was about 25% at 90 minutes. Considering that the drug dissolved in the human stomach will be continuously diluted and discharged into the intestine, the concentration of metformin in the stomach should be much lower than that of the immediate-release tablets.

In order to better understand the dissolution of the product in the human body, especially considering that the influence of gastrointestinal peristalsis mechanical force is more obvious when taking the medicine after a meal, the inventor conducted a reciprocating barrel dissolution test, using simulated postprandial gastric juice (the composition of postprandial gastric juice and the detection method of metformin are the same as above) as the medium, and setting the reciprocating barrel reciprocating rate to 30dpm. As a result, D2 began to rupture at 25 minutes, and the dissolution rate reached more than 85% in 60 minutes. E12 began to rupture at about 50 minutes, the dissolution rate was about 25% at 60 minutes, and the dissolution rate was about 40% at 90 minutes. E15 had a dissolution rate of about 15% at 60 minutes and about 35% at 90 minutes. The experiment further shows that it is difficult to achieve the "enteric" effect when taking D2 after a meal.

When taking medicine during or after a meal, the drug often stays in the stomach longer than when taking medicine on an empty stomach, and the pH value is also higher, which can reach 4.5-5.0. According to the analysis of the pharmacokinetic and BA trial results, metformin enteric-coated tablets taken during meals can stay in the stomach for up to several hours, so the dissolution in pH4.5 medium is of great value to this product. The results of clinical and simulated postprandial gastric juice tests showed that when the dissolution in pH4.5 medium exceeded 20% (D2), the intragastric release increased significantly, and the adverse reactions also increased significantly, while when it was less than 15% (E12, E15), limited intragastric release could be achieved, and when it was less than 7.5 % (E4, E13, E22), it could achieve bioequivalence with the best target product (E1).

At present, the requirements of drug regulatory authorities in most countries for enteric-coated tablets are that the dissolution in a medium of pH 1.0 is less than 5 %~ 10% in 2 hours. D2 meets this requirement, which shows that this requirement may be difficult to ensure that the enteric effect is achieved when taking the medicine after meals. Since ordinary preparations of metformin hydrochloride (i.e., immediate release preparations) are taken after meals or during meals, many patients still habitually take metformin hydrochloride enteric-coated tablets after meals or during meals, so ensuring "enteric effect " after meals is more important for metformin hydrochloride. At the same time, products which can be taking before meals, after meals or during meals can significantly improve patients' compliance.

D5 were significantly improved in upper gastrointestinal reactions compared with those of Glucophage, but the lower gastrointestinal reactions were higher than those of Glucophage, D2 and E1. The incidence of lower gastrointestinal adverse events (mainly diarrhea) of D5 was 130% of that of ordinary tablets, and if the duration of diarrhea was calculated, it was 156% of that of ordinary tablets. In addition, 5 patients in D5 had severe watery stools, 2 of which occurred after taking ordinary metformin tablets and worsened after switching to D5, and 3 have no diarrhea when taking ordinary metformin tablets and diarrhea appeared after switching to D5. The researchers judged that this symptom was an adverse reaction caused by the drug.

Since metformin is highly irritating to the gastrointestinal mucosa, the inventors consider that the watery stools is caused by drug irritating the colorectal mucosa. According to the drug formulation and dissolution, D2 should be released in the stomach, duodenum and upper small intestine (depending on the time of drug administration), and E1 should be released in the small intestine. The small intestine has fast peristalsis and a large amount of intestinal fluid, so the drug is quickly diluted and has a weak irritation to the colon. D5 dissolves slowly and is likely to be released in the colon. The colon has slow peristalsis, less fluid, and less drug absorption, which may form a high concentration locally and irritate the colon mucosa.

### Pharmacokinetic studies and results

In order to study the absorption of metformin enteric-coated preparations with different release characteristics, as well as their relationship with hypoglycemic effects and adverse reactions, the inventors conducted a pharmacokinetic study of the preparations.

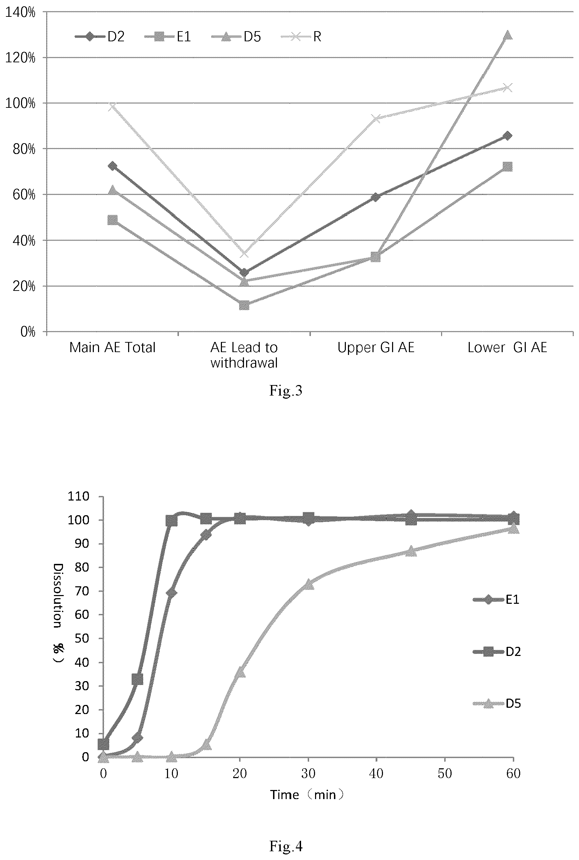

Four products with different release characteristics in the above clinical trials were selected, namely, three metformin hydrochloride enteric-coated tablets E1, D2, and D5 (the dissolution curves are shown in Figure 4. Considering that the drug dissolved in acid will also be absorbed, D2 is the cumulative dissolution. Cumulative dissolution = dissolution + dissolution at 0 hour or dissolution in acid) and the reference preparation (metformin hydrochloride tablets, trade name: Glucophage^{®}) , and a pharmacokinetic study was conducted in healthy subjects, and the bioavailability of E1, D2, and D5 relative to Glucophage (R) was calculated.

### Method:

Inclusion criteria: subjects must meet all of the following criteria:
1) Healthy subjects: medical history, vital signs, physical examination, laboratory tests, electrocardiogram, chest X- ray and other related examinations are within the normal range, or the researchers judge that the abnormalities have no clinical significance.
2) Men and women aged 18 to 65 (including borderline values).
3) Male weight ≥50 kg, female weight ≥45 kg, body mass index between 19 and 28 (including the critical value) [ body mass index (BMI) = weight (kg) / height² (m²) ] ;

Before statistical analysis, if any of the following situations occurs, a comprehensive judgment will be made on whether to exclude the subject based on factors such as the period of the trial and the reason for withdrawal:
1) If the first sample is C ₘₐₓ and no early samples (within 30 minutes after administration) are collected, the pharmacokinetic data of the corresponding period of the subject will not be included in the evaluation;
2) If a subject vomits within twice the mediumn Tmax after administration, the pharmacokinetic data of the subject in the corresponding period will not be included in the evaluation.
3) If the plasma concentration before administration (0 -hour concentration) is greater than 5% of ₜₕₑ Cmax after administration, the pharmacokinetic data of the corresponding period of the subject will not be included in the evaluation.
4) Trial data may have large deviations or the PK parameters may not be calculated due to reasons such as violation of the dosing regimen, incorrect blood collection time, improper sample handling or storage, and poor subject compliance.

Dosage schedule: a four-period crossover design was used, with administration once on the first day of each period and a three-day washout period during the two-week period. Twenty subjects were randomly divided into four sequences at a ratio of 1:1:1:1. Subjects fasted for 10 h before administration. On the day of administration, the reference preparation (R / Glucophage, 1 tablet, 500 mg) or the test preparation (E1, D2, D5 ; all 500 mg) was taken fasting with 240 ml of water. A standard lunch was eaten 4 h after taking the medicine.

The study design is shown in Table 9.

**Table 9: Study design**

| | **Period 1** | **Period 2** | **Period 3** | **Period 4** |
|---|---|---|---|---|
| Sequence 1 | R | D2 | E1 | D5 |
| Sequence 2 | D5 | R | D2 | E1 |
| Sequence 3 | E1 | D5 | R | D2 |
| Sequence 4 | D2 | E1 | D5 | R |

During the entire trial, the subjects ate standard meals provided by the research center, subjects avoided strenuous exercise and long-term lie in bed, and were prohibited from taking juice, tea, coffee, alcohol and other caffeinated or ethanol beverages, and smoking. If adverse events occurred during the trial, the researchers were required to follow up the subjects until the adverse events were alleviated or the symptoms disappeared.

Blood was collected at 0 h (within 60 min before drug administration) and 0.5 h, 1.0 h, 1.5 h, 2.0 h, 2.5 h, 3.0 h, 3.5 h, 4.0 h, 4.5 h, 5.0 h, 6.0 h, 7.0 h, 8.0 h, 10.0 h, 12.0 h, 15 h, and 24.0 h after taking the drug in each period (a total of 18 blood collection points each period, a total of 72 blood collection points in 4 period). 3 ml of peripheral venous blood was collected at each blood collection point and placed in a K2EDTA blood collection tube, centrifuged (1700 g, 2°C~8°C, set temperature at 4°C) for 10 min, and plasma was separated. The plasma samples were stored in a low-temperature refrigerator until detection. The drug concentration of metformin hydrochloride in plasma was determined by liquid chromatography - tandem mass spectrometry (HPLC-MS/MS) (Reference: Zhang D, Wang GC, Huang JQ, et al., LC-MS/MS determination of metformin in human plasma [ J ]. Chin J Pharm Anal (Journal of Drug Analysis), 2011, 31(2): 317-321; Zhang Dan, Post-marketing bioequivalence re-evaluation of metformin hydrochloride enteric-coated tablets in humans, Chinese Journal of Pharmaceutical Sciences, Vol. 47, No. 18, October 2012).

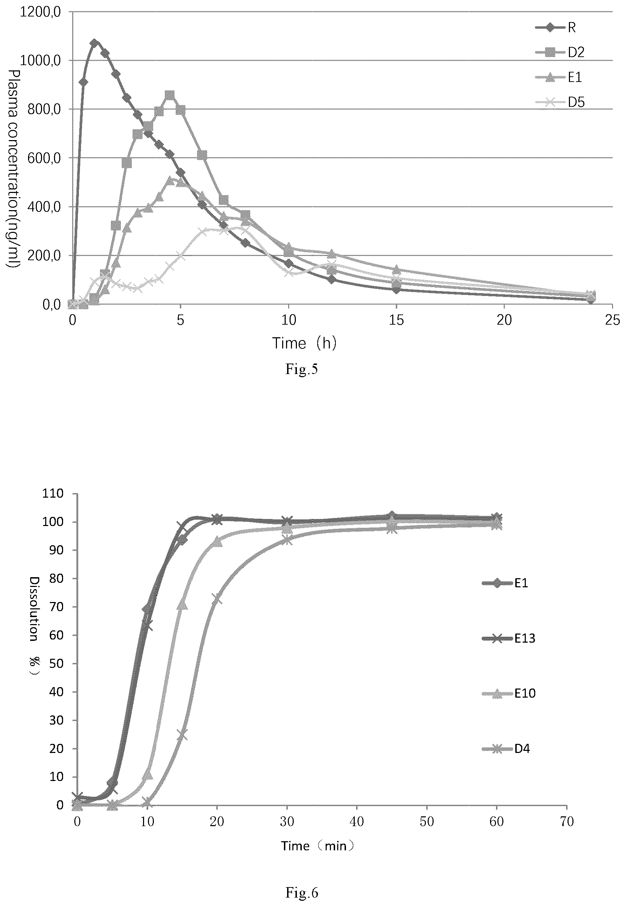

The results are as follows (Tables 10-11 and Figure 5).

**Table 10: Pharmacokinetic parameters (PKPS)**

| **Parameters (Mean±SD)** | **D2 (n=20)** | **E1 (n=18)** | **D5 (n=20)** | **R(n=19)** |
|---|---|---|---|---|
| C ₘₐₓ (ng/ml) | 982.99±254.50 | 710.51±299.89 | 567.26±399.85 | 1115.05±216.59 |
| AUC₀₋ₜ (h*ng/ml) | 5701.69±1235.03 | 4836.79±1636.07 | 3110.98±1614.21 | 6323.58±1333.26 |
| AUC_{0-∞}(h*ng/ml) | 5978.04±1217.67 | 5108.78±1716.44 | 3432.56±1614.64 | 6436.91±1375.07 |
| Tmax (h) | 4.50 ( 2.5-5.0 ) | 5.00 ( 3.0-12.0 ) | 7.00 ( 1.5-15.0 ) | 1.00 ( 0.5-2.0 ) |
| T _{1/2} (h) | 5.50±2.06 | 4.75±1.50 | 5.26±1.42 | 4.00±0.82 |

| | | | | |
|---|---|---|---|---|
| Note: Mean±SD represents the arithmetic mean ± standard deviation; T _{max is expressed} as mediumn (minimum value -- maximum value). | | | | |

**Table 11: Relative bioavailability**

| | **Pharmacokinetic parameters** | **Geometric mean (T)** | **Geometric mean (R)** | **( T/R ) %** | **90% confidence interval** |
|---|---|---|---|---|---|
| D2/R | C ₘₐₓ (ng/ml) | 946.78 | 1111.90 | 85.15 | (71.01 - 102.11) |
| | AUC ₀₋ₜ (h*ng/ml) | 5540.72 | 6304.65 | 87.88 | (77.92 - 99.12) |
| | AUC _{0-∞} (h*ng/ml) | 5829.38 | 6417.82 | 90.83 | (81.31 - 101.47) |
| E1/R | C ₘₐₓ (ng/ml) | 623.26 | 1111.90 | 56.05 | (46.46 - 67.63) |
| | AUC ₀₋ₜ (h*ng/ml) | 4353.32 | 6304.65 | 69.05 | (60.96 - 78.21) |
| | AUC _{0-∞} (h*ng/ml) | 4606.11 | 6417.82 | 71.77 | (64.00 - 80.49) |
| D5/R | C ₘₐₓ (ng/ml) | 457.86 | 1111.90 | 41.18 | (34.34 - 49.38) |
| | AUC ₀₋ₜ (h*ng/ml) | 2750.27 | 6304.65 | 43.62 | (38.68 - 49.20) |
| | AUC _{0-∞} (h*ng/ml) | 3097.01 | 6417.82 | 48.26 | (43.11 - 54.02) |

The results showed that the dissolution behavior of metformin enteric-coated tablets was highly correlated with absorption. The relative bioavailability of the metformin hydrochloride enteric-coated tablets with the fastest (D2), medium (E1) and slowest (D5) release relative to Glucophage was 91%, 72%, and 48%, respectively. Dissolution was highly correlated with absorption (see Figures 4 and 5). Combined with clinical trials, dissolution was also significantly correlated with clinical efficacy and adverse reactions.

There was one case of nausea in the reference preparation group and one case of diarrhea in D5, both of which recovered on the next day. No adverse reactions were found in the other groups.

The enteric-coated preparations disclosed in WO2013103384A and WO2013103919A have a relative bioavailability reduced by 20% to 60%, preferably 40% to 60%, compared with the immediate-release preparations, having the same amount of the biguanide compound. It can be seen that D5 meets the enteric-coated preparations of metformin hydrochloride described in the aforementioned patent documents. Consistent with the results of a 16-week clinical study conducted by Elcelyx (Robert R. Henry et al.), D5 (bioavailability reduced by 52%) did not achieve the desired clinical efficacy. On the contrary, a relative bioavailability of 60 % to 80 % (E1) produced the best efficacy and the lowest adverse reactions.

### In vivo and in vitro correlation studies and results

The above clinical trials and pharmacokinetic studies of E1, D2 and D5 show that the efficacy, safety and absorption of metformin enteric-coated preparations are significantly correlated with drug dissolution. To further study the relationship between drug release characteristics and drug absorption, efficacy and safety, the inventors conducted the following in vitro and in vivo correlation studies.

### Trial 1:

To study the effects of enteric coating materials, weight gain and related release characteristics on drug absorption, the following representative formulations are selected:
Example 1 (E1): used as a reference preparation (93.8% dissolution in pH 6.8 medium in 15 minutes and 101.1 % dissolution in 20 minutes).

Example 10 (E10): The tablet core is the same as E1, and the enteric coating material is hydroxypropyl methylcellulose phthalate (HP-50) with a onset dissolving point of pH 5.0. The weight gain in coating is 8%. The dissolution in pH 6.8 medium is 71.1 % in 15 minutes and 93.3 % in 20 minutes.

Example 13 (E13): The tablet core is the same as E1, and the enteric coating material is methacrylic acid methyl methacrylate copolymer (Eutrac L100) with a onset dissolving point of pH 6.0. The weight gain in coating is 3.5%. The dissolution in pH 6.8 medium is 95.6 % in 15 minutes and 98.1 % in 20 minutes.

Comparative Example 4 (D4): The tablet core is the same as E1, and the copolymer of methacrylic acid methyl methacrylate (Eutrac L100) with a onset dissolving point of pH 6.0 is used as the enteric coating material. The weight gain in coating is 6.5%, and the dissolution rate in pH 6.8 medium is 25.0 % in 15 minutes and 73.0% in 20 minutes.

E1, E10, E13 and D4 in pH 6.8 medium are shown in Figure 6 (where D2 is the cumulative dissolution).

The study design is shown in Table 12.

**Table 12: Study design**

| **Trial 1** | Period 1 | Period **2** | Period 3 | Period 4 |
|---|---|---|---|---|
| Sequence 1 | E1 | E10 | E13 | D4 |
| Sequence 2 | D4 | E1 | E10 | E13 |
| Sequence 3 | E13 | D4 | E1 | E10 |
| Sequence 4 | E10 | E13 | D4 | E1 |

Because the previous pharmacokinetic trial showed that the plasma concentration was still high at 24 hours, this trial extended the blood collection time to 48 hours (adding two blood collection points at 36 hours and 48 hours), and other methods (inclusion criteria, dosing regimen, plasma concentration detection method, etc.) were the same as the pharmacokinetic trial.

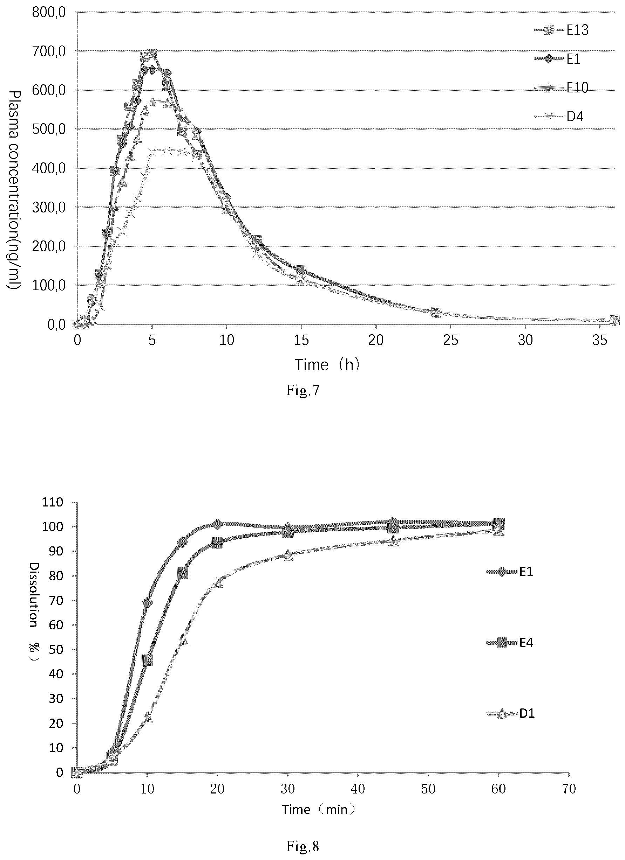

**Results:** see Tables 13, 14 and Figure 7.

**Table 13: Pharmacokinetic parameters ( PKPS )**

| Parameters (Mean±SD) | **E10 (n=20)** | **E13 (n=20)** | **D4 (n=19)** | **E1 (n=20)** |
|---|---|---|---|---|
| C ₘₐₓ (ng/ml) | 864.46±191.19 | 898.64±213.11 | 724.08±230.87 | 910.68±203.74 |
| AUC₀₋ₜ (h*ng/ml) | 5705.69±1459.71 | 6329.51±1271.74 | 4959.86±1256.55 | 6382.01±1233.42 |
| AUC_{0-∞} (h*ng/ml) | 5765.44±1458.68 | 6399.42±1288.50 | 5008.66±1259.49 | 6463.54±1259.21 |
| Tmax (h) | 5.00 (2.50-10.00) | 4.50 (2.50-12.00) | 6.00 (1.50-10.00) | 5.00 (1.50-8.00) |
| T _{1/2} (h) | 7.53±3.44 | 7.57±2.38 | 7.42±1.95 | 7.85±3.90 |

| | | | | |
|---|---|---|---|---|
| Note: Mean±SD represents the arithmetic mean ± standard deviation; Tₘₐₓ is expressed as mediumn (minimum value - maximum value). | | | | |

**Table 14: Relative bioavailability**

| | **Pharmacokinetic parameters** | **Geometric mean (T)** | **Geometric mean (E1)** | **( T/E1 ) %** | **90% confidence interval** |
|---|---|---|---|---|---|
| E13/E1 | C ₘₐₓ (ng/ml) | 872.10 | 886.86 | 98.34 | (90.14 - 107.28) |
| | AUC ₀₋ₜ (h*ng/ml) | 6209.89 | 6264.48 | 99.13 | (91.40 - 107.51) |
| | AUC _{0-∞} (h*ng/ml) | 6278.26 | 6342.85 | 98.98 | (91.25 - 107.37) |
| D4/E1 | C ₘₐₓ (ng/ml) | 694.99 | 886.86 | 78.36 | (71.72 - 85.62) |
| | AUC ₀₋ₜ (h*ng/ml) | 4823.05 | 6264.48 | 76.99 | (70.88 - 83.62) |
| | AUC _{0-∞} (h*ng/ml) | 4871.39 | 6342.85 | 76.80 | (70.70 - 83.43) |
| E10/E1 | C ₘₐₓ (ng/ml) | 846.18 | 886.86 | 95.41 | (87.46 - 104.09) |
| | AUC ₀₋ₜ (h*ng/ml) | 5544.65 | 6264.48 | 88.51 | (81.61 - 96.00) |
| | AUC _{0-∞} (h*ng/ml) | 5605.27 | 6342.85 | 88.37 | (81.47 - 95.86) |

E13 is bioequivalent to E1, with a BA (relative bioavailability) of about 99%. Although E10 is bioequivalent to E1, its BA is only 88% of that of E1. D4 has significantly reduced absorption, with a BA of only 77%, and is not bioequivalent to E1. The trial results further show that the in vitro dissolution (especially the dissolution in a pH 6.8 medium) of the metformin hydrochloride enteric-coated tablets of the present invention is well consistent with its in vivo absorption.

One adverse reaction (diarrhea) occurred on D4, and no adverse reactions were found in other groups.

### Trial 2:

To study the effects of disintegration rate of the tablet core and related release characteristics on drug absorption, the following representative formulations were selected:
Example 1 (E1): used as a reference preparation (R; Disintegration time of tablet core is 2-4 minutes. Dissolution is 93.8% in pH 6.8 medium within 15 minutes.)

Example 4 (E4): Enteric coating is the same as E1. Disintegration time of tablet core is 6 to 8 minutes. Dissolution is 81.3 % in pH 6.8 medium in 15 minutes, and 93.6 % in 20 minutes.

Comparative Example 1 (D1) : Enteric coating was the same as E1. Disintegration time of tablet core is 12-15 minutes. Dissolution is 54.3% in pH 6.8 medium in 15 minutes, and 77.7% in 20 minutes.

The dissolution curve in pH 6.8 medium is shown in Figure 8.

The study design is shown in Table 15.

**Table 15: study Design**

| **Trial 2** | Period 1 | Period **2** | Period 3 |
|---|---|---|---|
| Sequence 1 | E1 | E4 | D1 |
| Sequence 2 | D1 | E1 | E4 |
| Sequence 3 | E4 | D1 | E1 |

The trial methods (inclusion and exclusion criteria, dosing regimen, plasma concentration detection method, etc.) are the same as those in Trial 1.

**Results:** See Tables 16, 17 and Figure 9.

**Table 16: Pharmacokinetic parameters ( PKPS )**

| **Parameters ( Mean±SD )** | **D1(n=18)** | **E4 (n=18)** | **E1 (n=18)** |
|---|---|---|---|
| Cₘₐₓ (ng/ml) | 718.52±145.13 | 850.39±248.99 | 882.52±231.72 |
| AUC ₀₋ₜ (h*ng/ml) | 4703.92±1076.55 | 5617.34±1590.36 | 5990.16±1652.69 |
| AUC _{0-∞} (h*ng/ml) | 4752.18±1083.80 | 5674.26±1606.71 | 6054.96±1667.50 |
| Tmax (h) | 6.00 ( 3.00-12.00 ) | 5.00 ( 2.00-12.00 ) | 4.50 ( 2.50-7.00 ) |
| T_{1/2} (h) | 8.17±2.47 | 9.06±2.39 | 8.25±2.53 |

| | | | |
|---|---|---|---|
| Note: Mean±SD represents the arithmetic mean ± standard deviation; Tₘₐₓ is expressed as mediumn (minimum value - maximum value). | | | |

E4 is bioequivalent to E1, but its BA is only 94% of that of E1. D1 is not bioequivalent to E1, and its BA is only about 80% of that of E1. The trial results show that the disintegration time of the tablet core has a significant effect on the dissolution and absorption of the drug.

No adverse reactions were observed in any group.

**Table 17: Relative bioavailability**

| | **Pharmacokinetic parameters** | **Geometric mean (T)** | **Geometric mean (E1)** | **( T/E1 ) %** | **90% confidence interval** |
|---|---|---|---|---|---|
| D1/E1 | C ₘₐₓ (ng/ml) | 704.12 | 848.44 | 82.99 | (74.01 - 93.06) |
| | AUC ₀₋ₜ (h*ng/ml) | 4580.00 | 5753.38 | 79.61 | (73.50 - 86.22) |
| | AUC _{0-∞} (h*ng/ml) | 4627.87 | 5816.59 | 79.56 | (73.41 - 86.24) |
| E4/E1 | C ₘₐₓ (ng/ml) | 816.50 | 848.44 | 96.24 | (85.83 - 107.91) |
| | AUC ₀₋ₜ (h*ng/ml) | 5394.94 | 5753.38 | 93.77 | (86.58 - 101.56) |
| | AUC _{0-∞} (h*ng/ml) | 5449.70 | 5816.59 | 93.69 | (86.44 - 101.55) |

The absorption site of metformin hydrochloride is the upper part of the small intestine. It is generally believed that the dissolution in pH 6.0 medium better reflects the dissolution in the upper part of the small intestine, and its dissolution is an important factor affecting the absorption and efficacy of this type of drug. However, the present invention surprisingly found that the absorption of this product is mainly related to the dissolution in pH 6.8 medium. The pharmaceutical industry often uses dissolution characteristics to distinguish the differences in formulation and processes and hopes to predict in vivo absorption or bioavailability (BA) based on dissolution behavior. Compared with BA, dissolution testing is much simpler and faster. If the correlation between dissolution and BA can be established, it will be of great value for selecting appropriate product formulation and processes and predicting drugability.

The above clinical trials, pharmacokinetic studies (E1, D2, D5) and in vitro and in vivo correlation studies (E1, E10, E13, D4 ; E1, D1, E4) all show that the in vivo absorption of enteric-coated metformin hydrochloride preparations has a good correlation with their in vitro dissolution (especially dissolution in pH 6.8 buffer) . Rapid dissolution in pH 6.8 buffer (more than 85% dissolution in 20 minutes, especially more than 85% dissolution in 15 minutes) and limited release in pH 4.5 medium (less than 7.5-15 % release in 2 hours) can achieve the best therapeutic effect and the lowest adverse reactions. Its bioavailability is about 60-80% relative to metformin hydrochloride IR preparations (such as Glucophage) .

The research of the present invention shows that there is a close correlation between the disintegration time of the tablet core, the onset dissolving point of the coating material and the weight gain in coating for the metformin hydrochloride enteric-coated tablets. The selected disintegration time of the tablet core, the onset dissolving point of the enteric coating material and the weight gain in coating can obtain metformin hydrochloride enteric-coated tablets with the above-mentioned specific drug release characteristics, resulting in better therapeutic effects and lower adverse reactions.

## Claims

1. A metformin hydrochloride enteric-coated tablet, comprising a tablet core containing a therapeutically effective amount of metformin hydrochloride and an enteric coating layer wrapping the tablet core, wherein the dissolution rate of metformin hydrochloride in a pH 4.5 medium within 2 hours is not higher than 15%, and the dissolution rate of metformin hydrochloride in a pH 6.8 medium within 20 minutes is not lower than 85%. Preferably, the dissolution rate of metformin hydrochloride in a pH 4.5 medium within 2 hours is not higher than 7.5%, and the dissolution rate of metformin hydrochloride in a pH 6.8 medium within 15 minutes is not lower than 85%.

2. The metformin hydrochloride enteric-coated tablets according to claim 1, **characterized in that** in the said enteric coating layer the onset dissolving point of the coating material is 5.0 to 6.0 by pH value and the weight gain in coating is 4.0-12.8 mg/cm². Preferably, the onset dissolving point of the coating material is 5.0 to 6.0 by pH value and the weight gain in coating is 4.8-9.6 mg/cm².

3. The metformin hydrochloride enteric-coated tablets according to claim 2, wherein the onset dissolving point of the coating material is 5.0 by pH value, and the weight gain in coating is 5.6-12.8 mg/cm².

4. The metformin hydrochloride enteric-coated tablets according to claim 2, wherein the onset dissolving point of the coating material is 5.5 by pH value, and the weight gain in coating is 4.8-11.2 mg/cm².

5. The metformin hydrochloride enteric-coated tablets according to claim 2, wherein the onset dissolving point of the coating material is 6.0 by pH value, and the weight gain in coating is 4.0-8.8 mg/cm².

6. The metformin hydrochloride enteric-coated tablets according to claims 1 to 5, wherein the coating material of the enteric coating layer is selected from one or more of the following: hydroxypropyl methylcellulose phthalate (HP-50), methacrylic acid ethyl acrylate copolymer (L100-55), methacrylic acid ethyl acrylate copolymer (L30D-55), hydroxypropyl methylcellulose phthalate (HP-55), hydroxypropyl methylcellulose acetate succinate (HPMCAS-L), methacrylic acid methyl methacrylate copolymer (L100 ; polyacrylic acid resin II), hydroxypropyl methylcellulose acetate succinate (HPMCAS-M), cellulose acetate phthalate (CAP).

7. The metformin hydrochloride enteric-coated tablets according to claims 1 to 6, wherein the disintegration time of the tablet core is no more than 10 minutes. Preferably, the disintegration time of the tablet core is no more than 5 minutes. More preferably, the disintegration time of the tablet core is 2-4 minutes.

8. The metformin hydrochloride enteric-coated tablets according to claims 1 to 7, wherein, if necessary, the tablet core is first coated with a separation layer before the enteric coating layer.

9. The metformin hydrochloride enteric-coated tablets according to claims 1 to 8, **characterized in that** the said tablets have a relative bioavailability of (60~80)% compared to a conventional preparation or a rapid-release preparation (such as Glucophage) containing the same content of metformin hydrochloride.

10. Uses of the metformin hydrochloride enteric-coated tablets according to claims 1 to 9 in the preparation of drugs for treating diseases. Preferably, the said disease includes but is not limited to diabetes and obesity.
